# EUROPEAN PATENT SPECIFICATION

(11) **EP 4 225 758 B1**
(45) Date of publication and mention of the grant of the patent: **26.11.2025**
(21) Application number: 21801775.4
(22) Date of filing: 08.10.2021
(51) Int. Cl.: C07D 487/20, A61P 35/00, A61K 31/519

(54) **SOLID STATE FORMS OF TRILACICLIB AND OF TRILACICLIB SALTS**
FESTE FORMEN VON TRILACICLIB UND TRILACICLIBSALZEN
FORMES À L'ÉTAT SOLIDE DE TRILACICLIB ET DE SELS DE TRILACICLIB

(30) Priority: 08.10.2020 IN 202011043937; 06.11.2020 IN 202011048631; 07.12.2020 IN 202011053223
(43) Date of publication of application: 16.08.2023
(73) Proprietor: Assia Chemical Industries Ltd., Tel Aviv 6944020 (IL)
(72) Inventor: MUTHUSAMY, Anantha Rajmohan, Sivakasi, Tamilnadu 626189 (IN); SINGH, Amit, Noida, Uttar Pradesh 201310 (IN); SOMASUNDARAM, Meenakshi Sundaram, Nagar DT, Tamilnadu 626203 (IN); WAGH, Yogesh Dhananjay, Thane, Maharashtra 421503 (IN); LUTHRA, Parven Kumar, Noida, Uttar Pradesh 201304 (IN)
(74) Representative: D Young & Co LLP
(86) International application number: PCT/US2021/054110
(87) International publication number: WO 2022/076779

(56) References cited:
- WO-A1-2012/061156
- WO-A1-2016/126889
- WO-A1-2018/005865
- WO-A1-2021/257587
- US-A1- 2020 123 168

## Description

### FIELD OF THE DISCLOSURE

The present disclosure encompasses solid state forms of Trilaciclib salts, processes for preparation thereof, and pharmaceutical compositions thereof.

### BACKGROUND OF THE DISCLOSURE

Trilaciclib, has the following chemical structure:

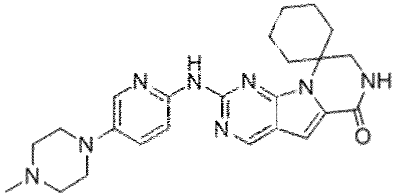

Trilaciclib is a small molecule with potential antineoplastic and chemoprotective activities. In particular, it is investigated for myelopreservation (preserving bone marrow function) in patients with small cell lung cancer (SCLC) that receive chemotherapy. Trilaciclib is also under clinical investigation for myelopreservation (i.e., reducing bone marrow suppression) in patients with non-small cell lung cancer (NSCLC), colorectal cancer (particularly metastatic colorectal cancer), breast cancer (particularly metastatic triple-negative breast cancer), and bladder cancer (particularly advanced/metastatic bladder cancer), that receive chemotherapy.

The compound is described in U.S. Patent No. 8,598,186; U.S. Patent No. 10,988,479 describes different polymorphs of Trilaciclib and Trilaciclib DiHCl; WO2018/005865 discloses a process for preparing pyrimidine-based compounds.

Polymorphism, the occurrence of different crystalline forms, is a property of some molecules and molecular complexes. A single molecule may give rise to a variety of polymorphs having distinct crystal structures and physical properties like melting point, thermal behaviors (e.g., measured by thermogravimetric analysis ("TGA"), or differential scanning calorimetry ("DSC")), X-ray diffraction (XRD) pattern, infrared absorption fingerprint, and solid state (¹³C) NMR spectrum. One or more of these techniques may be used to distinguish different polymorphic forms of a compound.

Different salts and solid state forms (including solvated forms) of an active pharmaceutical ingredient may possess different properties. Such variations in the properties of different salts and solid state forms and solvates may provide a basis for improving formulation, for example, by facilitating better processing or handling characteristics, changing the dissolution profile in a favorable direction, or improving stability (polymorph as well as chemical stability) and shelf-life. These variations in the properties of different salts and solid state forms may also offer improvements to the final dosage form, for instance, if they serve to improve bioavailability. Different salts and solid state forms and solvates of an active pharmaceutical ingredient may also give rise to a variety of polymorphs or crystalline forms, which may in turn provide additional opportunities to assess variations in the properties and characteristics of a solid active pharmaceutical ingredient.

Discovering new solid state forms and solvates of a pharmaceutical product may yield materials having desirable processing properties, such as ease of handling, ease of processing, storage stability, and ease of purification or as desirable intermediate crystal forms that facilitate conversion to other polymorphic forms. New solid state forms of a pharmaceutically useful compound can also provide an opportunity to improve the performance characteristics of a pharmaceutical product. It enlarges the repertoire of materials that a formulation scientist has available for formulation optimization, for example by providing a product with different properties, including a different crystal habit, higher crystallinity, or polymorphic stability, which may offer better processing or handling characteristics, improved dissolution profile, or improved shelf-life (chemical/physical stability). For at least these reasons, there is a need for additional solid state forms (including solvated forms) of Trilaciclib and of Trilaciclib salts.

### SUMMARY OF THE DISCLOSURE

The present disclosure provides crystalline Trilaciclib citrate salt, processes for preparation thereof, and pharmaceutical compositions thereof, as defined in the claims. The Trilaciclib citrate salt can be used to prepare other forms of Trilaciclib, Trilaciclib salts, or solvates of Trilaciclib or Trilaciclib salts.

The present disclosure provides crystalline Trilaciclib citrate salt, for use in the preparation of pharmaceutical compositions and/or formulations for use in medicine, in embodiments for the treatment of myelosuppression during chemotherapy, particularly for reducing chemotherapy-induced bone marrow suppression in patients with SCLC, NSCLC, colorectal cancer (particularly metastatic colorectal cancer), breast cancer (particularly metastatic triple-negative breast cancer), or bladder cancer (particularly advanced/metastatic bladder cancer); and particularly SCLC.

The present disclosure provides crystalline Trilaciclib citrate salt, for use in medicine, including as a chemoprotective agent (in particular; in patients suffering from: SCLC, colorectal cancer, breast cancer (particularly metastatic triple-negative breast cancer), and bladder cancer (particularly advanced/metastatic bladder cancer), and particularly SCLC, that receive chemotherapy), particularly for reducing chemotherapy-induced bone marrow suppression in patients with SCLC, NSCLC, colorectal cancer (particularly metastatic colorectal cancer), breast cancer (particularly metastatic triple-negative breast cancer), or bladder cancer (particularly advanced/metastatic bladder cancer); and particularly SCLC.

The present disclosure also encompasses the use of crystalline Trilaciclib citrate salt of the present disclosure for the preparation of pharmaceutical compositions and/or formulations.

In another aspect, the present disclosure provides pharmaceutical compositions comprising crystalline Trilaciclib citrate salt, according to the present disclosure.

The present disclosure includes processes for preparing the above mentioned pharmaceutical compositions. The processes include combining the crystalline Trilaciclib citrate salt, with at least one pharmaceutically acceptable excipient.

The crystalline Trilaciclib citrate salt, as defined herein and the pharmaceutical compositions or formulations of the crystalline Trilaciclib citrate salt, may be used as medicaments, such as for the treatment of myelosuppression during chemotherapy, particularly for reducing chemotherapy-induced bone marrow suppression in patients with SCLC, NSCLC, colorectal cancer (particularly metastatic colorectal cancer), breast cancer (particularly metastatic triple-negative breast cancer), or bladder cancer (particularly advanced/metastatic bladder cancer); and particularly SCLC.

The present disclosure also provides uses of crystalline Trilaciclib citrate salt of the present disclosure, or at least one of the above pharmaceutical compositions, for the manufacture of medicaments for treating, e.g., myelosuppression, particularly chemotherapy-induced myelosuppression, and particularly for reducing chemotherapy-induced bone marrow suppression in patients with SCLC, NSCLC, colorectal cancer (particularly metastatic colorectal cancer), breast cancer (particularly metastatic triple-negative breast cancer), or bladder cancer (particularly advanced/metastatic bladder cancer); and particularly SCLC.

In any embodiment of the present disclosure the patients or subjects may be receiving chemotherapy with any antineoplastic drug, particularly gemcitabine, docetaxel, fluorouracil, leucovorin, platinum-based chemotherapy drugs including carboplatin, and oxaliplatin, etoposide irinotecan, topotecan, or any combination of one or more thereof.

### BRIEF DESCRIPTION OF THE DRAWINGS

Figure 1 shows a characteristic X-ray powder diffraction pattern (XRPD) of Trilaciclib Form TT1.
Figure 2 shows a characteristic XRPD of Trilaciclib Form TT2.
Figure 3 shows a characteristic XRPD of Trilaciclib Form TT3.
Figure 4 shows a characteristic XRPD of Trilaciclib Form TT4.
Figure 5 shows a characteristic XRPD of Trilaciclib trifluoroacetate salt Form TTFA1.
Figure 6 shows a characteristic XRPD of Trilaciclib dihydrochloride salt Form THCl1.
Figure 7 shows a characteristic XRPD of Trilaciclib hydrochloride salt Form THCl.
Figure 8 shows a characteristic XRPD of Trilaciclib citrate salt Form TCT1.
Figure 9 shows a characteristic XRPD of Trilaciclib sulfate salt Form TS1.
Figure 10 shows a characteristic XRPD of Trilaciclib mesylate salt Form TMSA1.
Figure 11 shows a characteristic XRPD of Trilaciclib besylate salt Form TBSA1
Figure 12 shows a characteristic XRPD of Trilaciclib dihydrochloride salt Form THCl2.
Figure 13 shows a characteristic XRPD of Trilaciclib dihydrochloride salt Form THCl3.
Figure 14 shows a characteristic XRPD of Trilaciclib dihydrochloride salt Form THCl4.
Figure 15 shows a characteristic XRPD of Trilaciclib dihydrochloride salt Form THCl5.
Figure 16 shows a characteristic XRPD of Trilaciclib dihydrochloride salt Form THCl6.
Figure 17 shows a characteristic XRPD of Trilaciclib hydrobromide salt Form THBr1.

### DETAILED DESCRIPTION OF THE DISCLOSURE

The present disclosure encompasses crystalline Trilaciclib citrate salt, processes for preparation thereof, and pharmaceutical compositions thereof, as defined in the claims.

A solid state form (or polymorph) may be referred to herein as polymorphically pure or as substantially free of any other solid state (or polymorphic) forms. As used herein in this context, the expression "substantially free of any other forms" will be understood to mean that the solid state form contains about 20% (w/w) or less, about 10% (w/w) or less, about 5% (w/w) or less, about 2% (w/w) or less, about 1% (w/w) or less, or about 0% of any other forms of the subject compound as measured, for example, by XRPD. Thus, a crystalline polymorph of Trilaciclib, Trilaciclib salts, or a solvate of Trilaciclib or Trilaciclib salts, described herein as substantially free of any other solid state forms would be understood to contain greater than about 80% (w/w), greater than about 90% (w/w), greater than about 95% (w/w), greater than about 98% (w/w), greater than about 99% (w/w), or about 100% of the subject crystalline polymorph of Trilaciclib, Trilaciclib salt, or solvates of Trilaciclib or Trilaciclib salt. In some embodiments of the disclosure, the described crystalline polymorph of Trilaciclib, Trilaciclib salt, or solvate of Trilaciclib or Trilaciclib salt, may contain from about 1% to about 20% (w/w), from about 5% to about 20% (w/w), or from about 5% to about 10% (w/w) of one or more other crystalline polymorph of Trilaciclib, Trilaciclib salt, and/or solvate of Trilaciclib or Trilaciclib salt.

Depending on which other crystalline polymorphs a comparison is made, the crystalline polymorphs of the present disclosure may have advantageous properties selected from at least one of the following: chemical purity, flowability, solubility, dissolution rate, morphology or crystal habit, stability, such as chemical stability as well as thermal and mechanical stability with respect to polymorphic conversion, stability towards dehydration and/or storage stability, low content of residual solvent, a lower degree of hygroscopicity, flowability, and advantageous processing and handling characteristics such as compressibility and bulk density.

The crystalline polymorphs of the present disclosure may, in particular, be stable to stress conditions such as: grinding, pressure, heating, and/or exposure to humidity, and may have advantageous solubility characteristics at physiologically relevant pH values.

A solid state form, such as a crystal form or an amorphous form, may be referred to herein as being characterized by graphical data "as depicted in" or "as substantially depicted in" a Figure. Such data include, for example, powder X-ray diffractograms and solid state NMR spectra. As is well-known in the art, the graphical data potentially provides additional technical information to further define the respective solid state form (a so-called "fingerprint") which cannot necessarily be described by reference to numerical values or peak positions alone. In any event, the skilled person will understand that such graphical representations of data may be subject to small variations, e.g., in peak relative intensities and peak positions due to certain factors such as, but not limited to, variations in instrument response and variations in sample concentration and purity, which are well known to the skilled person. Nonetheless, the skilled person would readily be capable of comparing the graphical data in the Figures herein with graphical data generated for an unknown crystal form and confirm whether the two sets of graphical data are characterizing the same crystal form or two different crystal forms. A crystal form of Trilaciclib or salt of Trilaciclib referred to herein as being characterized by graphical data "as depicted in" or "as substantially depicted in" a Figure will thus be to include any crystal forms of Trilaciclib and of Trilaciclib salt characterized with the graphical data having such small variations, as are well known to the skilled person, in comparison with the Figure.

As used herein, and unless stated otherwise, the term "anhydrous" in relation to crystalline forms of Trilaciclib, relates to a crystalline form of Trilaciclib which does not include any crystalline water (or other solvents) in a defined, stoichiometric amount within the crystal. Moreover, an "anhydrous" form would generally not contain more than 1% (w/w), of either water or organic solvents as measured for example by TGA.

The term "solvate," as used herein and unless indicated otherwise, refers to a crystal form that incorporates a solvent in the crystal structure. When the solvent is water, the solvate is often referred to as a "hydrate." The solvent in a solvate may be present in either a stoichiometric or in a non-stoichiometric amount.

As used herein, unless stated otherwise, the XRPD measurements are taken using copper Kα radiation wavelength 1.5418 Å. XRPD peaks reported herein are measured using CuK α radiation, λ = 1.5418 Å, typically at a temperature of 25 ± 3°C.

A thing, *e.g.,* a reaction mixture, may be characterized herein as being at, or allowed to come to "room temperature" or "ambient temperature", often abbreviated as "RT." This means that the temperature of the thing is close to, or the same as, that of the space, *e.g.,* the room or fume hood, in which the thing is located. Typically, room temperature is from about 20°C to about 30°C, or about 22°C to about 27°C, or about 25°C.

The amount of solvent employed in a chemical process, e.g., a reaction or crystallization, may be referred to herein as a number of "volumes" or "vol" or "V." For example, a material may be referred to as being suspended in 10 volumes (or 10 vol or 10V) of a solvent. In this context, this expression would be understood to mean milliliters of the solvent per gram of the material being suspended, such that suspending a 5 grams of a material in 10 volumes of a solvent means that the solvent is used in an amount of 10 milliliters of the solvent per gram of the material that is being suspended or, in this example, 50 mL of the solvent. In another context, the term "v/v" may be used to indicate the number of volumes of a solvent that are added to a liquid mixture based on the volume of that mixture. For example, adding solvent X (1.5 v/v) to a 100 ml reaction mixture would indicate that 150 mL of solvent X was added.

A process or step may be referred to herein as being carried out "overnight." This refers to a time interval, e.g., for the process or step, that spans the time during the night, when that process or step may not be actively observed. This time interval is from about 8 to about 20 hours, or about 10-18 hours, in some cases about 16 hours.

As used herein, the term "reduced pressure" refers to a pressure that is less than atmospheric pressure. For example, reduced pressure is about 10 mbar to about 50 mbar.

As used herein and unless indicated otherwise, the term "ambient conditions" refer to atmospheric pressure and a temperature of 22-24°C.

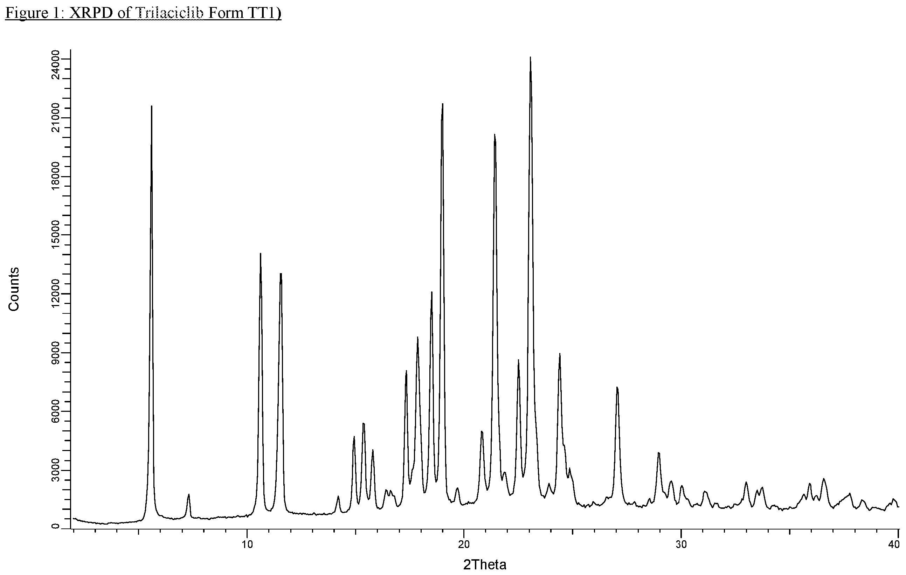

Disclosed is a crystalline polymorph of Trilaciclib, designated TT1. The crystalline Form TT1 of Trilaciclib may be characterized by data selected from one or more of the following: an X-ray powder diffraction pattern substantially as depicted in Figure 1; an X-ray powder diffraction pattern having peaks at 5.6, 10.7, 11.5, 17.4 and 23.1 degrees 2-theta ± 0.2 degrees 2-theta; and combinations of these data.

Crystalline Form TT1 of Trilaciclib may be further characterized by an X-ray powder diffraction pattern having peaks at 5.6, 10.7, 11.5, 17.4 and 23.1 degrees 2-theta ± 0.2 degrees 2-theta, and also having any one, two, three or four additional peaks selected from 18.5, 19.0, 21.5 and 24.4 degrees 2-theta ± 0.2 degrees 2-theta.

Crystalline Form TT1 of Trilaciclib may be alternatively characterized by an X-ray powder diffraction pattern having peaks at 5.6, 10.7, 11.5, 17.4, 18.5, 19.0, 21.5, 23.1, and 24.4 degrees 2-theta ± 0.2 degrees 2-theta.

Crystalline Form TT1 of Trilaciclib may be isolated.

Crystalline Form TT1 of Trilaciclib is anhydrous form.

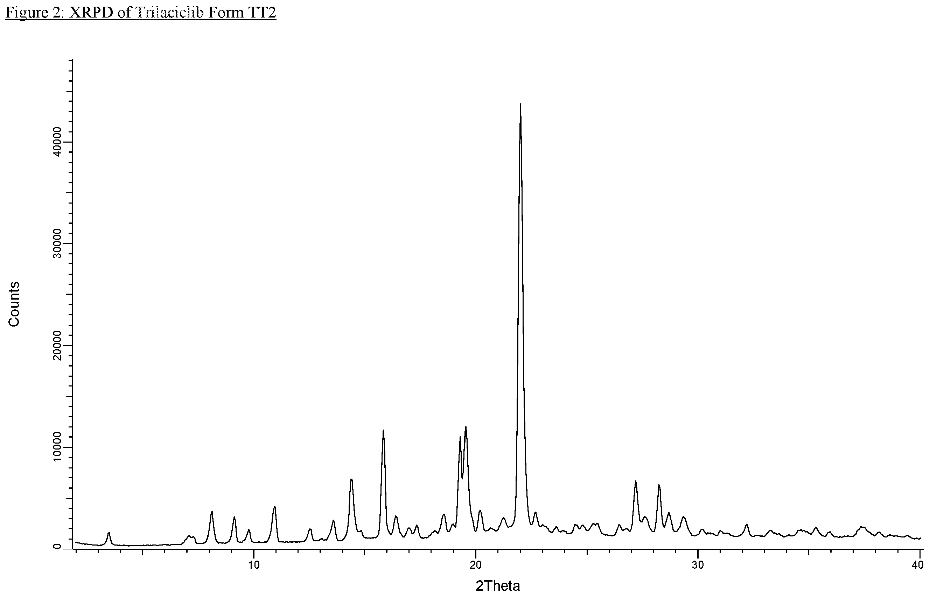

Disclosed is a crystalline polymorph of Trilaciclib, designated Form TT2. The crystalline Form TT2 of Trilaciclib may be characterized by data selected from one or more of the following: an X-ray powder diffraction pattern substantially as depicted in Figure 2; an X-ray powder diffraction pattern having peaks at 8.1, 9.1, 11.0, 14.5 and 15.9 degrees 2-theta ± 0.2 degrees 2-theta; and combinations of these data.

Crystalline Form TT2 of Trilaciclib may be further characterized by an X-ray powder diffraction pattern having peaks at 8.1, 9.1, 11.0, 14.5 and 15.9 degrees 2-theta ± 0.2 degrees 2-theta, and also having any one, two, three or four additional peaks selected from 19.6, 20.2, 22.1 and 27.2 degrees 2-theta ± 0.2 degrees 2-theta.

Crystalline Form TT2 of Trilaciclib may be alternatively characterized by an X-ray powder diffraction pattern having peaks at 8.1, 9.1, 11.0, 14.5, 15.9, 19.6, 20.2, 22.1, and 27.degrees 2-theta ± 0.2 degrees 2-theta.

Crystalline Form TT2 of Trilaciclib may be isolated.

Crystalline Form TT2 of Trilaciclib is acetic acid solvate. Alternatively, crystalline Form TT2 is a salt of Trilaciclib with acetic acid (i.e., Trilaciclib acetate salt).

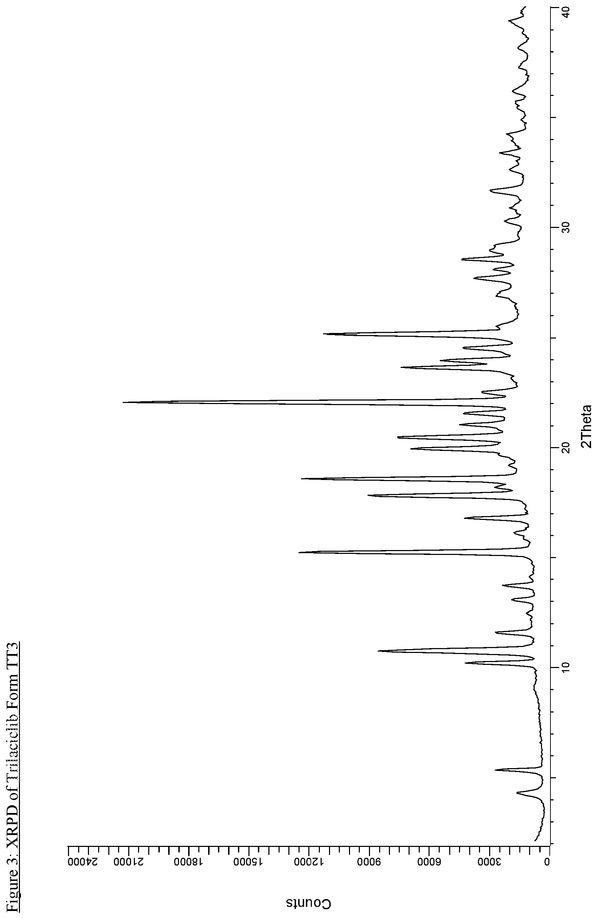

Disclosed is a crystalline polymorph of Trilaciclib, designated Form TT3. The crystalline Form TT3 of Trilaciclib may be characterized by data selected from one or more of the following: an X-ray powder diffraction pattern substantially as depicted in Figure 3; an X-ray powder diffraction pattern having peaks at 5.3, 10.2, 10.8, 17.8 and 25.2 degrees 2-theta ± 0.2 degrees 2-theta; and combinations of these data.

Crystalline Form TT3 of Trilaciclib may be further characterized by an X-ray powder diffraction pattern having peaks at 5.3, 10.2, 10.8, 17.8 and 25.2 degrees 2-theta ± 0.2 degrees 2-theta, and also having any one, two, three or four additional peaks selected from 15.3, 18.6, 23.6 and 24.0 degrees 2-theta ± 0.2 degrees 2-theta.

Crystalline Form TT3 of Trilaciclib may be alternatively characterized by an X-ray powder diffraction pattern having peaks at 5.3, 10.2, 10.8, 15.3, 17.8, 18.6, 23.6, 24.0, and 25.2 degrees 2-theta ± 0.2 degrees 2-theta.

Crystalline Form TT3 of Trilaciclib may be isolated.

Crystalline Form TT3 of Trilaciclib is formic acid solvate. Alternatively, crystalline Form TT3 is a salt of Trilaciclib with formic acid (i.e., Trilaciclib formic salt). The molar ratio between Trilaciclib and formic acid may be 1:2.

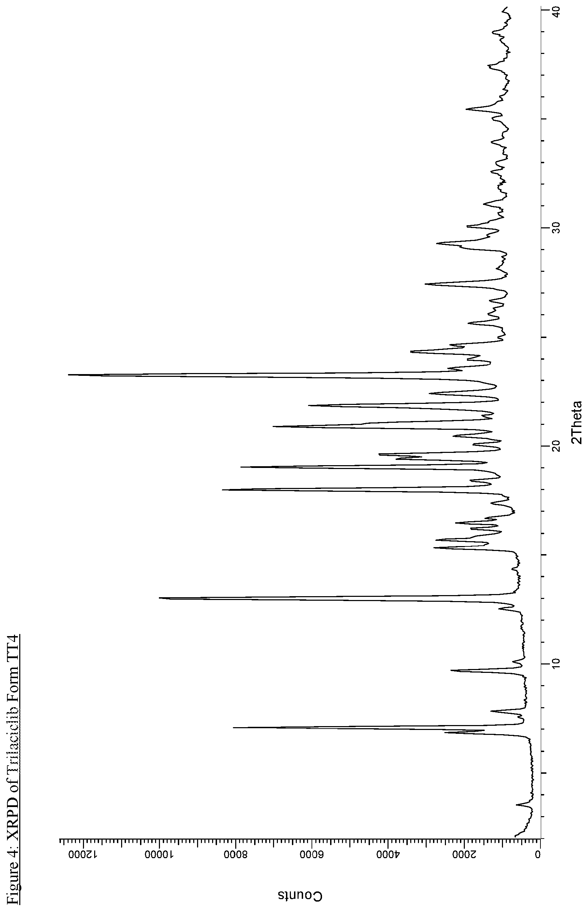

Disclosed is a crystalline polymorph of Trilaciclib, designated Form TT4. The crystalline Form TT4 of Trilaciclib may be characterized by data selected from one or more of the following: an X-ray powder diffraction pattern substantially as depicted in Figure 4; an X-ray powder diffraction pattern having peaks at 13.1, 18.1, 19.1, 20.9 and 23.3 degrees 2-theta ± 0.2 degrees 2-theta; and combinations of these data.

Crystalline Form TT4 of Trilaciclib may be further characterized by an X-ray powder diffraction pattern having peaks at 13.1, 18.1, 19.1, 20.9 and 23.3 degrees 2-theta ± 0.2 degrees 2-theta, and also having any one, two, three or four additional peaks selected from 7.1, 9.7, 22.4 and 27.4 degrees 2-theta ± 0.2 degrees 2-theta.

Crystalline Form TT4 of Trilaciclib may be alternatively characterized by an X-ray powder diffraction pattern having peaks at 7.1, 9.7, 13.1, 18.1, 19.1, 20.9, 22.4, 23.3, and 27.4 degrees 2-theta ± 0.2 degrees 2-theta.

Crystalline Form TT4 of Trilaciclib may be isolated.

Crystalline Form TT4 of Trilaciclib is propionic acid solvate. Alternatively, crystalline Form TT4 is a salt of Trilaciclib with propionic acid (i.e., Trilaciclib propionate salt).

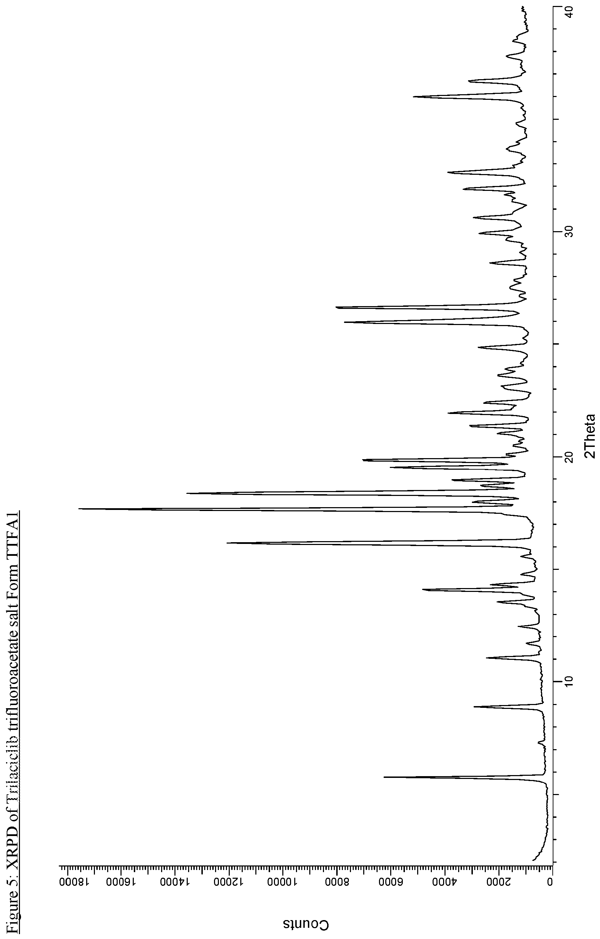

Disclosed is a crystalline polymorph of Trilaciclib trifluoroacetate salt; designated Form TTFA1. The crystalline Form TTFA1 may be characterized by data selected from one or more of the following: an X-ray powder diffraction pattern substantially as depicted in Figure 5; an X-ray powder diffraction pattern having peaks at 5.8, 16.2, 17.7, 18.4 and 26.7 degrees 2-theta ± 0.2 degrees 2-theta; and combinations of these data.

Crystalline Form TTFA1 of Trilaciclib trifluoroacetic acid salt may be further characterized by an X-ray powder diffraction pattern having peaks at 5.8, 16.2, 17.7, 18.4 and 26.7 degrees 2-theta ± 0.2 degrees 2-theta, and also having any one, two, three or four additional peaks selected from 8.9, 14.1, 26.0 and 36.0 degrees 2-theta ± 0.2 degrees 2-theta.

Crystalline Form TTFA1 of Trilaciclib trifluoroacetic acid salt may be alternatively characterized by an X-ray powder diffraction pattern having peaks at 5.8, 8.9, 14.1, 16.2, 17.7, 18.4, 26.0, 26.7, and 36.0 degrees 2-theta ± 0.2 degrees 2-theta.

Disclosed is a Trilaciclib hydrochloride salt, for example Trilaciclib monohydrochloride or Trilaciclib dihydrochloride. Advantageously, Trilaciclib monohydrochloride or Trilaciclib dihydrochloride may be crystalline.

Disclosed is crystalline Trilaciclib dihydrochloride.

Also disclosed are crystalline polymorphs of Trilaciclib dihydrochloride, which may be distinguished by X-ray powder diffraction patterns having characteristic peaks.

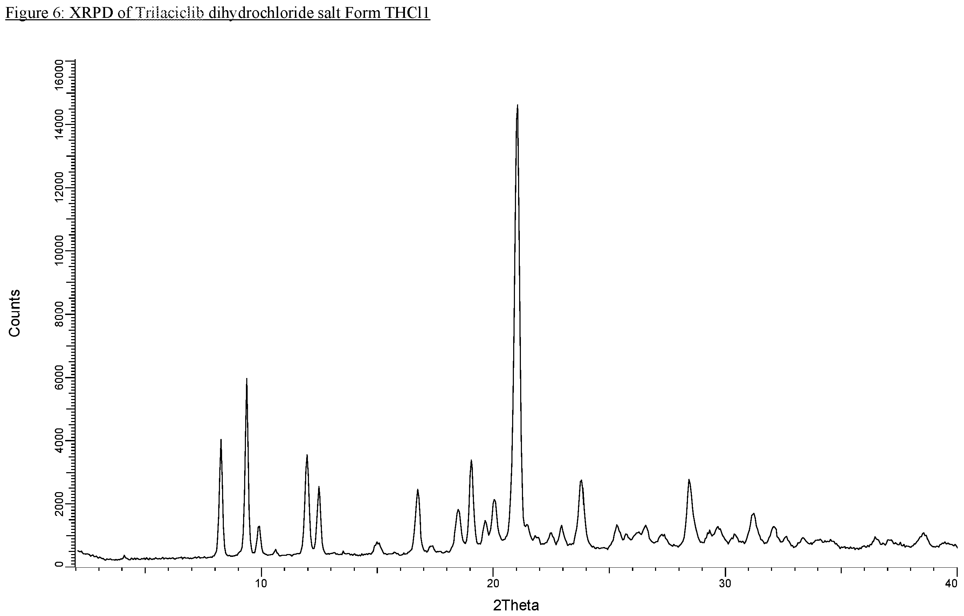

Disclosed is a crystalline polymorph of Trilaciclib dihydrochloride salt; designated Form THCl1. The crystalline Form THCI1_may be characterized by data selected from one or more of the following: an X-ray powder diffraction pattern substantially as depicted in Figure 6; an X-ray powder diffraction pattern having peaks at 8.3, 9.4, 12.0, 19.1 and 21.1 degrees 2-theta ± 0.2 degrees 2-theta; and combinations of these data.

Crystalline Form THCl1 of Trilaciclib dihydrochloride salt may be further characterized by an X-ray powder diffraction pattern having peaks at 8.3, 9.4, 12.0, 19.1 and 21.1 degrees 2-theta ± 0.2 degrees 2-theta, and also having any one, two, three or four additional peaks selected from 4.1, 10.0, 12.6 and 23.8 degrees 2-theta ± 0.2 degrees 2-theta.

Crystalline Form THCl1 of Trilaciclib dihydrochloride salt may be alternatively characterized by an X-ray powder diffraction pattern having peaks at 4.1, 8.3, 9.4, 10.0, 12.0, 12.6, 19.1, 21.1, and 23.8 degrees 2-theta ± 0.2 degrees 2-theta.

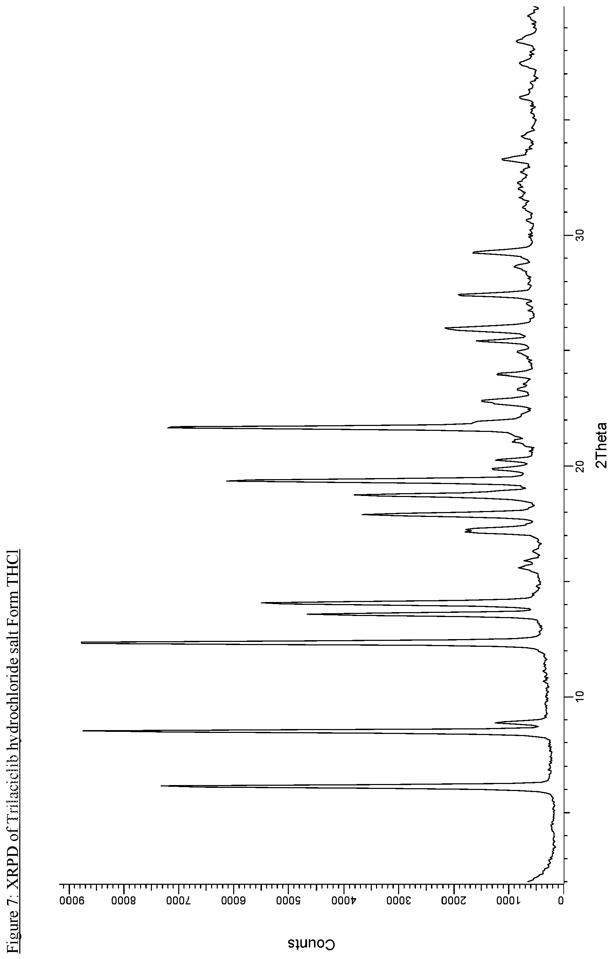

Disclosed is a crystalline polymorph of Trilaciclib hydrochloride salt; designated Form THCl. The crystalline Form THCl may be characterized by data selected from one or more of the following: an X-ray powder diffraction pattern substantially as depicted in Figure 7; an X-ray powder diffraction pattern having peaks at 6.2, 8.5, 13.6, 14.1 and 21.7 degrees 2-theta ± 0.2 degrees 2-theta; and combinations of these data.

Crystalline Form THCl of Trilaciclib hydrochloride salt may be further characterized by an X-ray powder diffraction pattern having peaks at 6.2, 8.5, 13.6, 14.1 and 21.7 degrees 2-theta ± 0.2 degrees 2-theta, and also having any one, two, three or four additional peaks selected from 12.3, 17.9, 18.7 and 19.4 degrees 2-theta ± 0.2 degrees 2-theta.

Crystalline Form THCl of Trilaciclib hydrochloride salt may be alternatively characterized by an X-ray powder diffraction pattern having peaks at 6.2, 8.5, 12.3, 13.6, 14.1, 17.9, 18.7, 19.4, and 21.7 degrees 2-theta ± 0.2 degrees 2-theta.

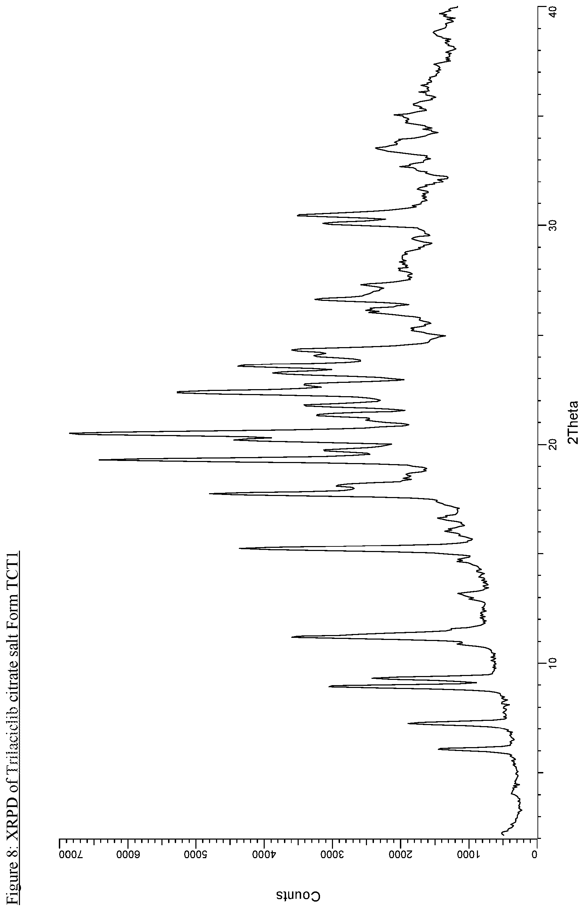

Disclosed is a crystalline polymorph of Trilaciclib citrate salt; designated Form TCT1. The crystalline Form TCT1 may be characterized by data selected from one or more of the following: an X-ray powder diffraction pattern substantially as depicted in Figure 8; an X-ray powder diffraction pattern having peaks at 6.1, 7.3, 11.3, 15.3 and 19.3 degrees 2-theta ± 0.2 degrees 2-theta; and combinations of these data.

Crystalline Form TCT1 of Trilaciclib citrate salt may be further characterized by an X-ray powder diffraction pattern having peaks at 6.1, 7.3, 11.3, 15.3 and 19.3 degrees 2-theta ± 0.2 degrees 2-theta, and also having any one, two, three or four additional peaks selected from 8.9, 9.3, 20.4 and 22.7 degrees 2-theta ± 0.2 degrees 2-theta.

Crystalline Form TCT1 of Trilaciclib citrate salt may be alternatively characterized by an X-ray powder diffraction pattern having peaks at 6.1, 7.3, 8.9, 9.3, 11.3, 15.3, 19.3, 20.4, and 22.7 degrees 2-theta ± 0.2 degrees 2-theta.

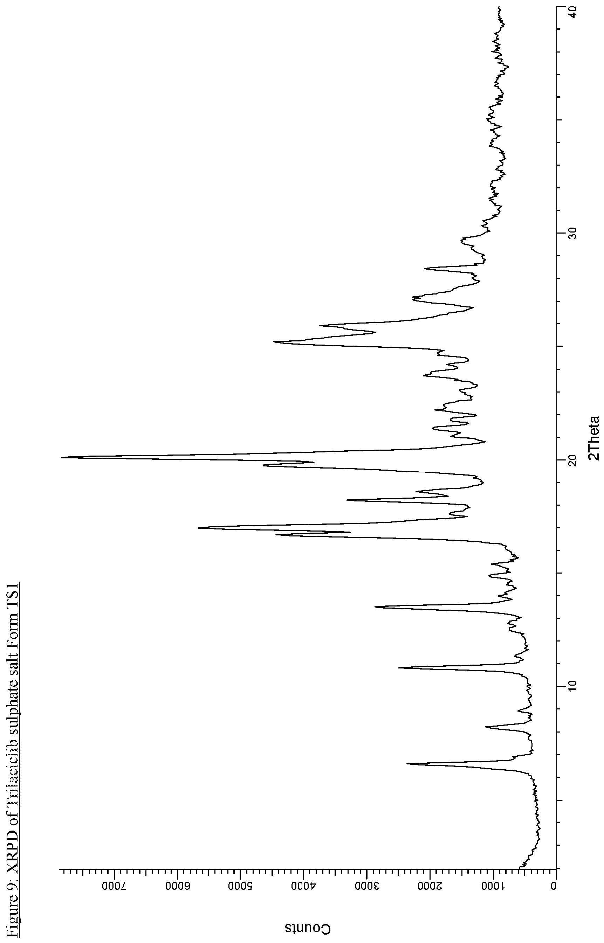

Disclosed is a crystalline polymorph of Trilaciclib sulphate salt; designated Form TS1. The crystalline Form TS1 may be characterized by data selected from one or more of the following: an X-ray powder diffraction pattern substantially as depicted in Figure 9; an X-ray powder diffraction pattern having peaks at 10.9, 13.5, 17.0, 20.1 and 25.2 degrees 2-theta ± 0.2 degrees 2-theta; and combinations of these data.

Crystalline Form TS1 of Trilaciclib sulphate salt may be further characterized by an X-ray powder diffraction pattern having peaks at 10.9, 13.5, 17.0, 20.1 and 25.2 degrees 2-theta ± 0.2 degrees 2-theta, and also having any one, two, three or four additional peaks selected from 6.6, 8.2, 18.2 and 18.6 degrees 2-theta ± 0.2 degrees 2-theta.

Crystalline Form TS1 of Trilaciclib sulphate salt may be alternatively characterized by an X-ray powder diffraction pattern having peaks at 6.6, 8.2, 10.9, 13.5, 17.0, 18.2, 18.6, 20.1, and 25.2 degrees 2-theta ± 0.2 degrees 2-theta.

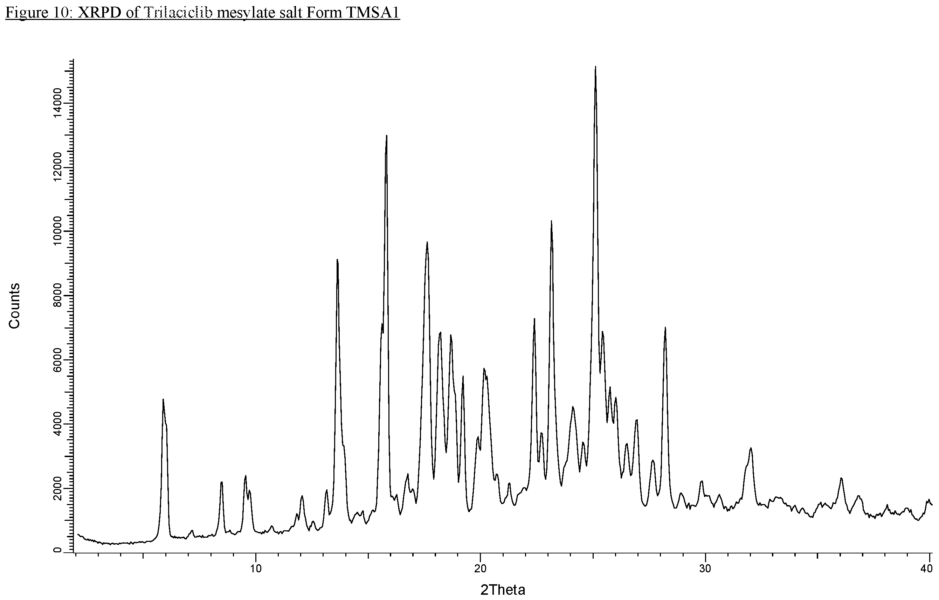

Disclosed is a crystalline polymorph of Trilaciclib mesylate salt; designated Form TMSA1. The crystalline Form TMSA1 may be characterized by data selected from one or more of the following: an X-ray powder diffraction pattern substantially as depicted in Figure 10; an X-ray powder diffraction pattern having peaks at 13.6, 15.8, 23.1, 25.1 and 28.2 degrees 2-theta ± 0.2 degrees 2-theta; and combinations of these data.

Crystalline Form TMSA1 of Trilaciclib mesylate salt may be further characterized by an X-ray powder diffraction pattern having peaks at 13.6, 15.8, 23.1, 25.1 and 28.2 degrees 2-theta ± 0.2 degrees 2-theta, and also having any one, two, three or four additional peaks selected from 6.0, 17.6, 18.2 and 22.4 degrees 2-theta ± 0.2 degrees 2-theta.

Crystalline Form TMSA1 of Trilaciclib mesylate salt may be alternatively characterized by an X-ray powder diffraction pattern having peaks at 6.0, 13.6, 15.8, 17.6, 18.2, 22.4, 23.1, 25.1, and 28.2 degrees 2-theta ± 0.2 degrees 2-theta.

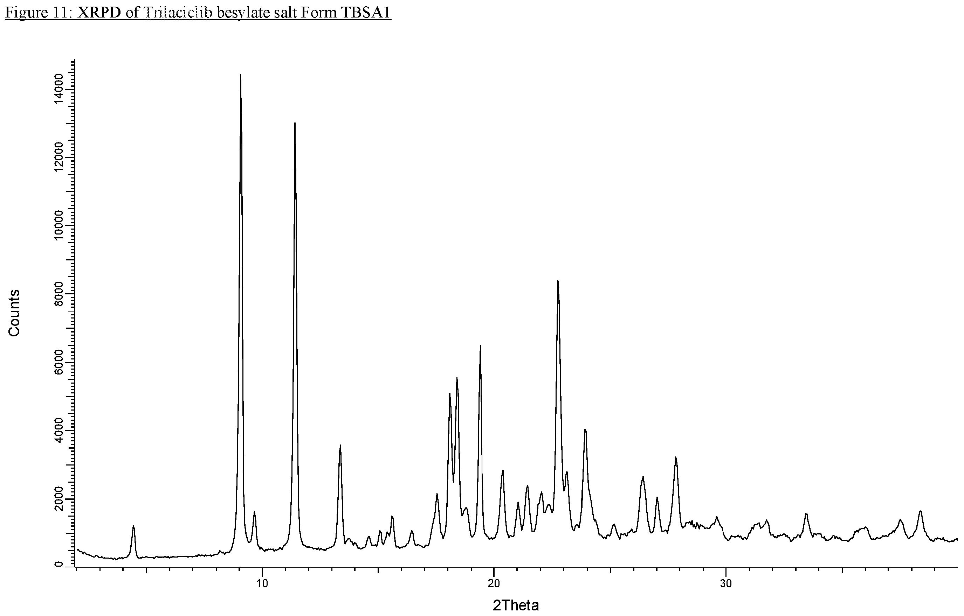

Disclosed is a crystalline polymorph of Trilaciclib besylate salt; designated Form TBSA1. The crystalline Form TBSA1 may be characterized by data selected from one or more of the following: an X-ray powder diffraction pattern substantially as depicted in Figure 11; an X-ray powder diffraction pattern having peaks at 9.1, 11.5, 13.4, 19.4 and 22.8 degrees 2-theta ± 0.2 degrees 2-theta; and combinations of these data.

Crystalline Form TBSA1 of Trilaciclib besylate salt may be further characterized by an X-ray powder diffraction pattern having peaks at 9.1, 11.5, 13.4, 19.4 and 22.8 degrees 2-theta ± 0.2 degrees 2-theta, and also having any one, two, three or four additional peaks selected from 18.1, 18.4, 20.4 and 23.9 degrees 2-theta ± 0.2 degrees 2-theta.

Crystalline Form TBSA1 of Trilaciclib besylate salt may be alternatively characterized by an X-ray powder diffraction pattern having peaks at 9.1, 11.5, 13.4, 18.1, 18.4, 19.4, 20.4, 22.8, and 23.9 degrees 2-theta ± 0.2 degrees 2-theta.

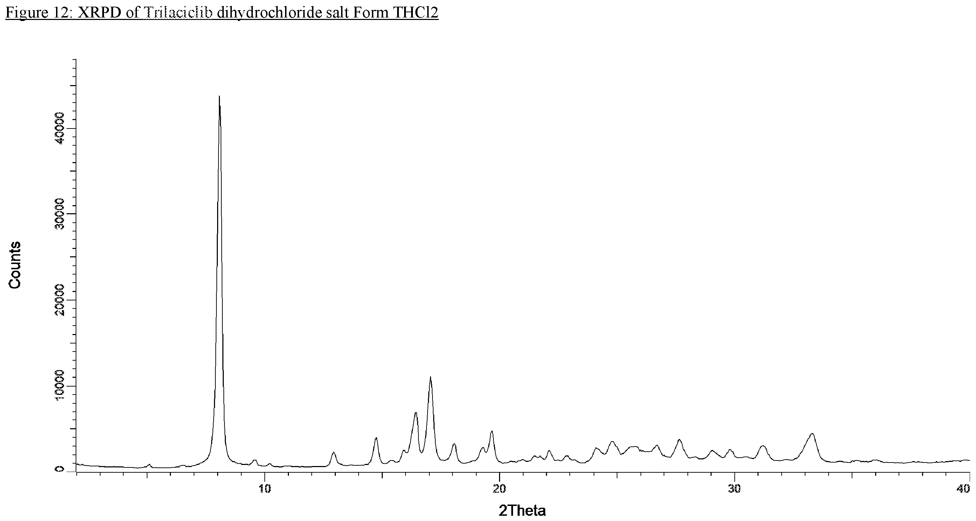

Disclosed is a crystalline polymorph of Trilaciclib dihydrochloride salt; designated Form THCl2.The crystalline Form THC12_may be characterized by data selected from one or more of the following: an X-ray powder diffraction pattern substantially as depicted in Figure 12; an X-ray powder diffraction pattern having peaks at 12.9, 16.3, 19.6, 27.6 and 33.2 degrees 2-theta ± 0.2 degrees 2-theta; and combinations of these data.

Crystalline Form THCl2 of Trilaciclib dihydrochloride salt may be further characterized by an X-ray powder diffraction pattern having peaks at 12.9, 16.3, 19.6, 27.6 and 33.2 degrees 2-theta ± 0.2 degrees 2-theta, and also having any one, two, three or four additional peaks selected from 8.0, 14.6, 17.0 and 18.0 degrees 2-theta ± 0.2 degrees 2-theta.

Crystalline Form THCl2 of Trilaciclib dihydrochloride salt may be alternatively characterized by an X-ray powder diffraction pattern having peaks at 8.0, 12.9, 14.6, 16.3, 17.0, 18.0, 19.6, 27.6, and 33.2 degrees 2-theta ± 0.2 degrees 2-theta.

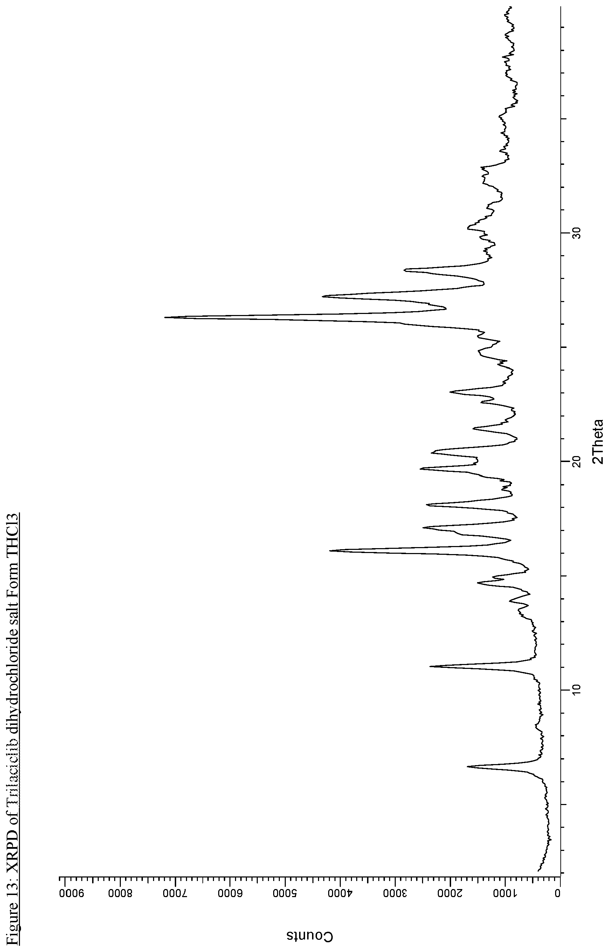

Disclosed is a crystalline polymorph of Trilaciclib dihydrochloride salt; designated Form THCl3. The crystalline Form THCl3_may be characterized by data selected from one or more of the following: an X-ray powder diffraction pattern substantially as depicted in Figure 13; an X-ray powder diffraction pattern having peaks at 6.6, 11.0, 16.1, 26.3 and 27.2 degrees 2-theta ± 0.2 degrees 2-theta; and combinations of these data.

Crystalline Form THCl3 of Trilaciclib dihydrochloride salt may be further characterized by an X-ray powder diffraction pattern having peaks at 6.6, 11.0, 16.1, 26.3 and 27.2 degrees 2-theta ± 0.2 degrees 2-theta, and also having any one, two, three or four additional peaks selected from 18.1, 20.5, 23.0 and 28.3 degrees 2-theta ± 0.2 degrees 2-theta.

Crystalline Form THCl3 of Trilaciclib dihydrochloride salt may be alternatively characterized by an X-ray powder diffraction pattern having peaks at 6.6, 11.0, 16.1, 18.1, 20.5, 23.0, 26.3, 27.2, and 28.3 degrees 2-theta ± 0.2 degrees 2-theta.

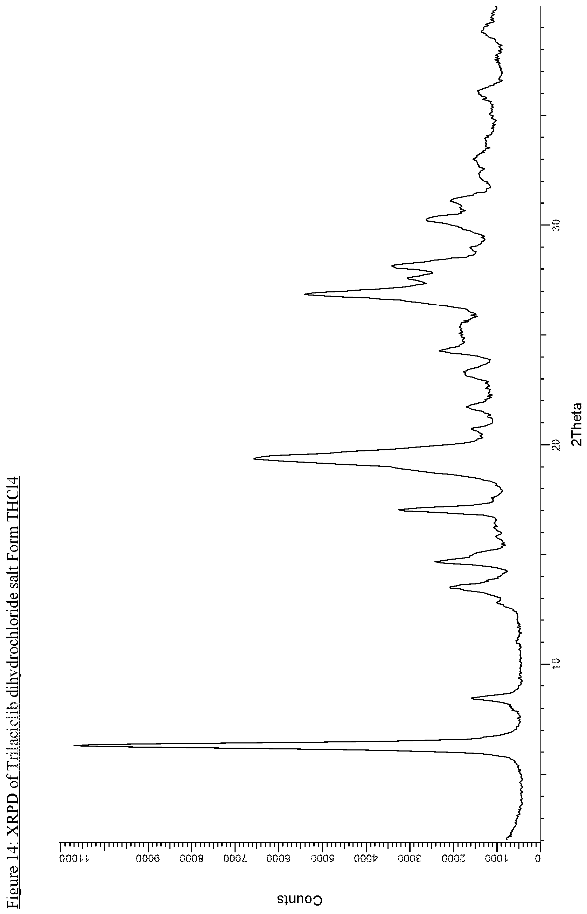

Disclosed is a crystalline polymorph of Trilaciclib dihydrochloride salt; designated Form THCl4.The crystalline Form THCl4_may be characterized by data selected from one or more of the following: an X-ray powder diffraction pattern substantially as depicted in Figure 14; an X-ray powder diffraction pattern having peaks at 6.3, 8.5, 13.5, 19.4 and 26.9 degrees 2-theta ± 0.2 degrees 2-theta; and combinations of these data.

Crystalline Form THCl4 of Trilaciclib dihydrochloride salt may be further characterized by an X-ray powder diffraction pattern having peaks at 6.3, 8.5, 13.5, 19.4 and 26.9 degrees 2-theta ± 0.2 degrees 2-theta, and also having any one, two, three or four additional peaks selected from 14.7, 17.1, 27.6 and 28.2 degrees 2-theta ± 0.2 degrees 2-theta.

Crystalline Form THCl4 of Trilaciclib dihydrochloride salt may be alternatively characterized by an X-ray powder diffraction pattern having peaks at 6.3, 8.5, 13.5, 14.7, 17.1, 19.4, 26.9, 27.6 and 28.2 degrees 2-theta ± 0.2 degrees 2-theta.

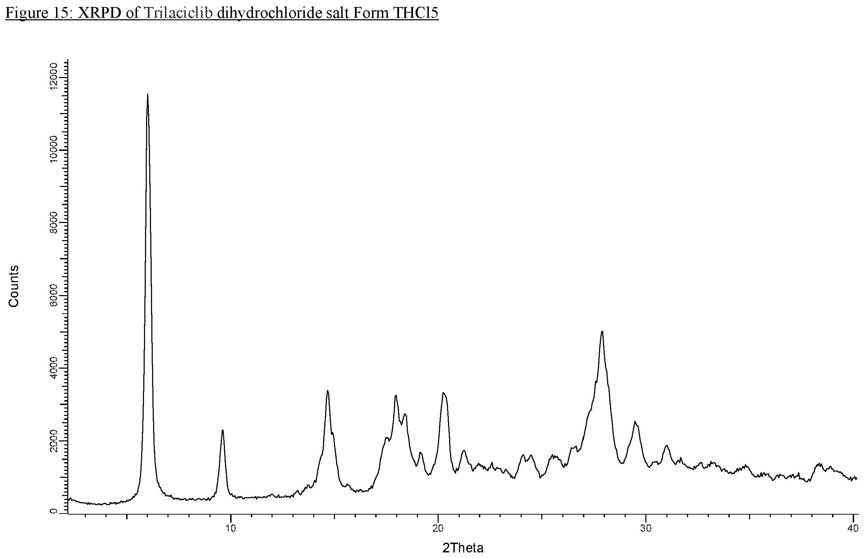

Disclosed is a crystalline polymorph of Trilaciclib dihydrochloride salt; designated Form THCl5. The crystalline Form THCl5 may be characterized by data selected from one or more of the following: an X-ray powder diffraction pattern substantially as depicted in Figure 15; an X-ray powder diffraction pattern having peaks at 6.1, 9.6, 14.7, 18.4 and 27.9 degrees 2-theta ± 0.2 degrees 2-theta; and combinations of these data.

Crystalline Form THCl5 of Trilaciclib dihydrochloride salt may be further characterized by an X-ray powder diffraction pattern having peaks at 6.1, 9.6, 14.7, 18.4 and 27.9 degrees 2-theta ± 0.2 degrees 2-theta, and also having any one, two or three additional peaks selected from 18.0, 20.3 and 29.5 degrees 2-theta ± 0.2 degrees 2-theta.

Crystalline Form THCl5 of Trilaciclib dihydrochloride salt may be alternatively characterized by an X-ray powder diffraction pattern having peaks at 6.1, 9.6, 14.7, 18.0, 18.4, 20.3, 27.9, and 29.5 degrees 2-theta ± 0.2 degrees 2-theta.

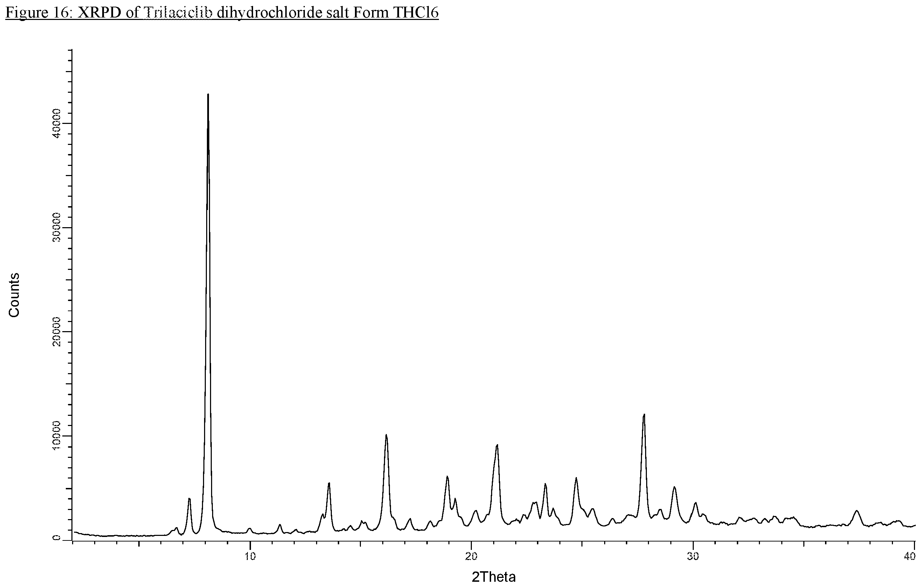

Disclosed is a crystalline polymorph of Trilaciclib dihydrochloride salt; designated Form THCl6. The crystalline Form THCl6 may be characterized by data selected from one or more of the following: an X-ray powder diffraction pattern substantially as depicted in Figure 16; an X-ray powder diffraction pattern having peaks at 7.3, 18.9, 23.3, 27.7 and 29.2 degrees 2-theta ± 0.2 degrees 2-theta; and combinations of these data.

Crystalline Form THCl6 of Trilaciclib dihydrochloride salt may be further characterized by an X-ray powder diffraction pattern having peaks at 7.3, 18.9, 23.3, 27.7 and 29.2 degrees 2-theta ± 0.2 degrees 2-theta, and also having any one, two, three or four additional peaks selected from 8.1, 13.5, 16.2 and 21.1 degrees 2-theta ± 0.2 degrees 2-theta.

Crystalline Form THCl6 of Trilaciclib dihydrochloride salt may be alternatively characterized by an X-ray powder diffraction pattern having peaks at 7.3, 8.1, 13.5, 16.2, 18.9, 21.1, 23.3, 27.7 and 29.2 degrees 2-theta ± 0.2 degrees 2-theta.

Crystalline Form THCl6 of Trilaciclib dihydrochloride salt may be prepared by combining a solution of Trilaciclib dihydrochloride in a solvent with an antisolvent. The solvent may be selected from alcohols, water, and mixtures thereof.

Preferably, the solvent is a mixture of alcohols (preferably a C₁ to C₄ alcohol, and more particularly methanol) and water. The ratio of alcohol:water, preferably methanol: water may be from: about 4:1 to about 1:4, about 3.5:1 to about 1:3.5, about 3:1 to about 3:1 to about 1:3; about 2.5:1 to about 1:1.5, about 2.4:1 to about 1:1, or about 2.4:1 to about 2.3:1

The antisolvent may be an ether, an ester or an alkane. The ether may be a C₄₋₈ ether, and more preferably methyl tert-butyl ether (MTBE) or diisopropyl ether. The alkane may be C₅₋₈ straight or branched alkane, and more preferably heptane or hexane. The ester may be C2-5 ester; preferably ethyl acetate. The antisolvent may be an ether, particularly a C₄₋₈ ether, and more particularly methyl tert-butyl ether.

The antisolvent may be added to the solution, or the solution may be added to the antisolvent (reverse addition). Preferably, the solution is added to the antisolvent. The antisolvent may be pre-cooled prior to combining with the solution. The solution may be added slowly to the antisolvent, for example, the solvent may be added dropwise to the antisolvent. Particularly, the solvent may be added dropwise to a precooled antisolvent. The pre-cooled antisolvent may be at a temperature of: about - 5°C to about 15°C, about -2°C to about 10°C, or about 0°C to about 5°C. Optionally, the mixture is stirred during the addition.

The antisolvent: solvent ratio (v/v) may be: about 15:1 to about 1:5, about 10:1 to about 1:5, about 8:1 to about 1:2, about 5:1 to about 1:1, about 4:1 to about 2:1, about 3.5:1 to about 3:1 or about 3:1.

Following combining the solution with the antisolvent, the mixture may be stirred, optionally with cooling. Preferably, the cooling is to: about -5°C to about 15°C, about -2°C to about 10°C, or about 0°C to about 5°C. The stirring may be for any suitable period of time, optionally for about 0.5 hours to about 10 hours, about 0.7 hours to about 8 hours, about 1 hour to about 6 hours, or about 2 hours to about 4 hours.

The process may further comprise isolating the Trilaciclib dihydrochloride Form THCl6 by any suitable procedure, such as centrifugation, decantation, or filtration, optionally filtration. Following isolation, the product may be dried. Preferably the drying is under reduced pressure. The drying may be carried out at elevated temperature, for example a temperature of: about 30°C to about 120°C, about 40°C to about 100°C, about 50°C to about 80°C, about 50°C to about 70°C, about 55°C to about 65°C, or about 60°C. The drying may be for any suitable period of time, for example, about 6 hours to about 48 hours, about 10 hours to about 40 hours, about 18 to about 30 hours, about 20 to about 28 hours, or about 24 hours.

Crystalline form THCl6 of Trilaciclib dihydrochloride salt can be prepared by stirring Trilaciclib dihydrochloride in an organic solvent (preferably wherein the organic solvent is, or comprises, a mixture of acetonitrile and an alcohol, more particularly wherein the alcohol is a C₁₋₆ alcohol, particularly methanol, ethanol or isopropanol, and especially isopropanol. The vol/vol ratio of acetonitrile to alcohol (preferably isopropanol) may be: about 200:1 to about 10:1, about 180:1 to about 50:1, about 150:1 to about 60:1, about 120:1 to about 80:1, about 110:1 to about 90:1, or about 100: 1. The mixture may be stirred at a temperature of: about -5°C to about 15°C, about -2°C to about 10°C, or about 0°C to about 5°C. The stirring may be for any suitable period of time to prepare Form THCl6 of Trilaciclib dihydrochloride salt. Particularly, the stirring may be for a period of time of: about 6 hours to about 48 hours, about 10 hours to about 40 hours, about 15 hours to about 30 hours, about 18 hours to about 24 hours, or about 20 hours. The mixture of Trilaciclib dihydrochloride in a solvent comprising acetonitrile and an alcohol as described above, may be prepared by combining Trilaciclib in a solution with acetonitrile, with a solution of hydrochloric acid in the alcohol (preferably isopropanol). The hydrochloric acid may be in the form of hydrogen chloride in alcohol solvent, more particularly a C₁₋₆ alcohol, and especially isopropanol. Preferably, the hydrochloric acid is added to the Trilaciclib solution. Following the addition, the mixture may be stirred as described above. The process may further comprise isolating the Trilaciclib dihydrochloride Form THCl6 by any suitable procedure, such as centrifugation, decantation, or filtration, optionally filtration. Following isolation, the product may be dried. Preferably the drying is under reduced pressure. The drying may be carried out at elevated temperature, for example a temperature of: about 18°C to about 50°C, about 20°C to about 40°C, about 22°C to about 30°C, or about 25°C. The drying may be for any suitable period of time, for example, about 2 to about 60 minutes, about 5 to about 30 minutes, about 10 to about 15 minutes.

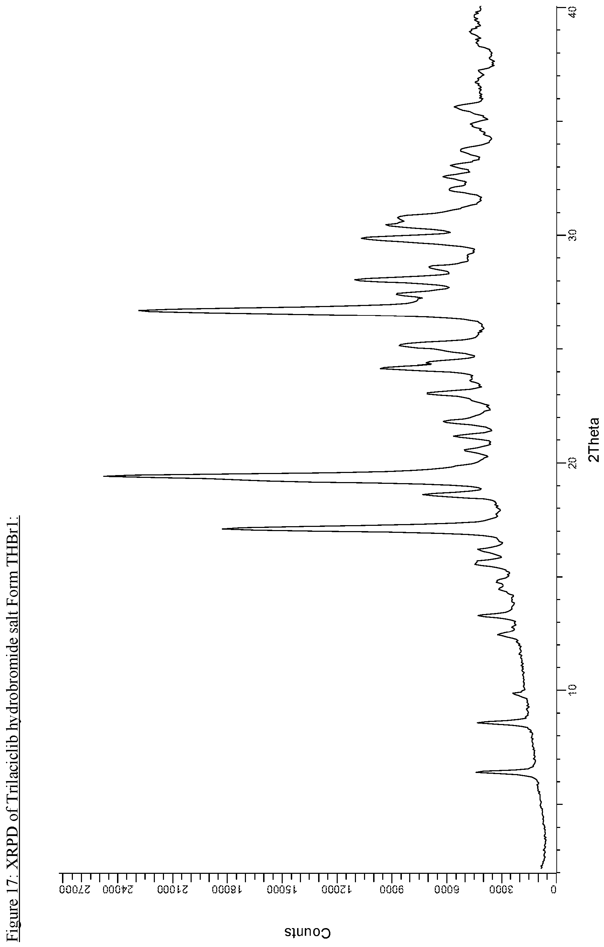

Disclosed is a crystalline polymorph of Trilaciclib hydrobromide salt; designated Form THBr1. The crystalline Form THBr1 may be characterized by data selected from one or more of the following: an X-ray powder diffraction pattern substantially as depicted in Figure 17; an X-ray powder diffraction pattern having peaks at 6.5, 8.6, 17.2, 19.5 and 26.8 degrees 2-theta ± 0.2 degrees 2-theta; and combinations of these data.

Crystalline Form THBr1 of Trilaciclib hydrobromide salt may be further characterized by an X-ray powder diffraction pattern having peaks at 6.5, 8.6, 17.2, 19.5 and 26.8 degrees 2-theta ± 0.2 degrees 2-theta, and also having any one, two, three or four additional peaks selected from 18.7, 25.2, 28.1 and 29.9 degrees 2-theta ± 0.2 degrees 2-theta.

Crystalline Form THBr1 of Trilaciclib hydrobromide salt may be further characterized by an X-ray powder diffraction pattern having peaks at 6.5, 8.6, 17.2, 18.7, 19.5, 25.2, 26.8, 28.1, and 29.9 degrees 2-theta ± 0.2 degrees 2-theta.

Any of the solid state forms of Trilaciclib, Trilaciclib salts, or solvates of Trilaciclib or Trilaciclib salts, described herein may be polymorphically pure or may be substantially free of any other solid state forms of the subject Trilaciclib, Trilaciclib salts, or solvates of Trilaciclib or Trilaciclib salts (for example a crystalline Trilaciclib which is polymorphically pure, may be substantially free of any other solid state forms of Trilaciclib; a crystalline Trilaciclib salt which is polymorphically pure, may be substantially free of any other solid state form of the Trilaciclib salt). In any aspect or embodiment of the present disclosure, any of the solid state forms of Trilaciclib, Trilaciclib salts, or solvates of Trilaciclib or Trilaciclib salts, described in any aspect or embodiment disclosed herein, may contain: about 20% (w/w) or less, about 10% (w/w) or less, about 5% (w/w) or less, about 2% (w/w) or less, about 1% (w/w) or less, about 0.5% (w/w) or less, about 0.2% (w/w) or less, about 0.1% (w/w) or less, or about 0%, of any other solid state forms of the subject compound (i.e. Trilaciclib, Trilaciclib salts, or solvates of Trilaciclib or Trilaciclib salts, respectively), preferably as measured by XRPD. Thus, any of the disclosed crystalline forms of Trilaciclib, Trilaciclib salts, or solvates of Trilaciclib or Trilaciclib salts, described herein may be substantially free of any other solid state forms of the subject Trilaciclib, Trilaciclib salts, or solvates of Trilaciclib or Trilaciclib salts, respectively, and may contain greater than about 80% (w/w), greater than about 90% (w/w), greater than about 95% (w/w), greater than about 98% (w/w), greater than about 99% (w/w), or about 100% of the subject solid state form of the Trilaciclib, Trilaciclib salts, or solvates of Trilaciclib or Trilaciclib salts, respectively.

The above crystalline polymorphs of Trilaciclib and of Trilaciclib salts can be used to prepare other crystalline polymorphs of Trilaciclib, other Trilaciclib salts, solvates of Trilaciclib or Trilaciclib salts, and solid state forms thereof.

The crystalline forms as described herein may be in the form of a solvate, or a hydrate, or a mixed solvate-hydrate. For example, a Trilaciclib hydrochloride may be in the form of a hydrate, i.e., the disclosure encompasses Trilaciclib hydrochlorides, such as Trilaciclib dihydrochloride, or the specific Trilaciclib hydrochlorides denoted herein as THCl1-THCl6, may be in the form of a hydrate. The hydrate may be a hemi-, mono-, di-, tri-, tetra-, penta-, or hexa-hydrate. Higher hydrates (e.g., tri-, tetra-, penta-, or hexa-) may be preferred. For example, crystalline Trilaciclib dihydrochloride, or the specific crystalline forms of Trilaciclib dihydrochloride denoted herein as THCl1-THCl6, may be in the form of a hydrate, including a pentahydrate.

The present disclosure provides crystalline Trilaciclib citrate salt, for use in the preparation of pharmaceutical compositions including Trilaciclib, Trilaciclib salts, and solvates of Trilaciclib or Trilaciclib salts and/or crystalline polymorphs thereof.

The present disclosure also encompasses the use of crystalline Trilaciclib citrate, of the present disclosure for the preparation of pharmaceutical compositions of crystalline polymorphs Trilaciclib, Trilaciclib salts, and solvates of Trilaciclib or Trilaciclib salts, and/or crystalline polymorphs thereof.

The present disclosure includes processes for preparing the above-mentioned pharmaceutical compositions. The processes include combining any one or a combination of the crystalline Trilaciclib citrate salt of the present disclosure with at least one pharmaceutically acceptable excipient.

Pharmaceutical combinations or formulations of the present disclosure contain crystalline Trilaciclib citrate, of the present disclosure. In addition to the active ingredient, the pharmaceutical formulations of the present disclosure can contain one or more excipients. Excipients are added to the formulation for a variety of purposes.

Diluents increase the bulk of a solid pharmaceutical composition, and can make a pharmaceutical dosage form containing the composition easier for the patient and caregiver to handle. Diluents for solid compositions include, for example, microcrystalline cellulose (e.g., Avicel^{®}), microfine cellulose, lactose, starch, pregelatinized starch, calcium carbonate, calcium sulfate, sugar, dextrates, dextrin, dextrose, dibasic calcium phosphate dihydrate, tribasic calcium phosphate, kaolin, magnesium carbonate, magnesium oxide, maltodextrin, mannitol, polymethacrylates (e.g., Eudragit^{®}), potassium chloride, powdered cellulose, sodium chloride, sorbitol, and talc.

Solid pharmaceutical compositions that are compacted into a dosage form, such as a tablet, can include excipients whose functions include helping to bind the active ingredient and other excipients together after compression. Binders for solid pharmaceutical compositions include acacia, alginic acid, carbomer (e.g. carbopol), carboxymethylcellulose sodium, dextrin, ethyl cellulose, gelatin, guar gum, hydrogenated vegetable oil, hydroxyethyl cellulose, hydroxypropyl cellulose (e.g. Klucel^{®}), hydroxypropyl methyl cellulose (e.g. Methocel^{®}), liquid glucose, magnesium aluminum silicate, maltodextrin, methylcellulose, polymethacrylates, povidone (e.g. Kollidon^{®}, Plasdone^{®}), pregelatinized starch, sodium alginate, and starch.

The dissolution rate of a compacted solid pharmaceutical composition in the patient's stomach can be increased by the addition of a disintegrant to the composition. Disintegrants include alginic acid, carboxymethylcellulose calcium, carboxymethylcellulose sodium (e.g., Ac-Di-Sol^{®}, Primellose^{®}), colloidal silicon dioxide, croscarmellose sodium, crospovidone (e.g., Kollidon^{®}, Polyplasdone^{®}), guar gum, magnesium aluminum silicate, methyl cellulose, microcrystalline cellulose, polacrilin potassium, powdered cellulose, pregelatinized starch, sodium alginate, sodium starch glycolate (e.g., Explotab^{®}), and starch.

Glidants can be added to improve the flowability of a non-compacted solid composition and to improve the accuracy of dosing. Excipients that can function as glidants include colloidal silicon dioxide, magnesium trisilicate, powdered cellulose, starch, talc, and tribasic calcium phosphate.

When a dosage form such as a tablet is made by the compaction of a powdered composition, the composition is subjected to pressure from a punch and dye. Some excipients and active ingredients have a tendency to adhere to the surfaces of the punch and dye, which can cause the product to have pitting and other surface irregularities. A lubricant can be added to the composition to reduce adhesion and ease the release of the product from the dye. Lubricants include magnesium stearate, calcium stearate, glyceryl monostearate, glyceryl palmitostearate, hydrogenated castor oil, hydrogenated vegetable oil, mineral oil, polyethylene glycol, sodium benzoate, sodium lauryl sulfate, sodium stearyl fumarate, stearic acid, talc, and zinc stearate.

Flavoring agents and flavor enhancers make the dosage form more palatable to the patient. Common flavoring agents and flavor enhancers for pharmaceutical products that can be included in the composition of the present disclosure include maltol, vanillin, ethyl vanillin, menthol, citric acid, fumaric acid, ethyl maltol, and tartaric acid.

Solid and liquid compositions can also be dyed using any pharmaceutically acceptable colorant to improve their appearance and/or facilitate patient identification of the product and unit dosage level.

In liquid pharmaceutical compositions of the present invention, Trilaciclib and Trilacicilib salt, and any other solid excipients can be dissolved or suspended in a liquid carrier such as water, vegetable oil, alcohol, polyethylene glycol, propylene glycol, or glycerin.

Liquid pharmaceutical compositions can contain emulsifying agents to disperse uniformly throughout the composition an active ingredient or other excipient that is not soluble in the liquid carrier. Emulsifying agents that can be useful in liquid compositions of the present invention include, for example, gelatin, egg yolk, casein, cholesterol, acacia, tragacanth, chondrus, pectin, methyl cellulose, carbomer, cetostearyl alcohol, and cetyl alcohol.

Liquid pharmaceutical compositions of the present invention can also contain a viscosity enhancing agent to improve the mouth-feel of the product and/or coat the lining of the gastrointestinal tract. Such agents include acacia, alginic acid bentonite, carbomer, carboxymethylcellulose calcium or sodium, cetostearyl alcohol, methyl cellulose, ethylcellulose, gelatin guar gum, hydroxyethyl cellulose, hydroxypropyl cellulose, hydroxypropyl methyl cellulose, maltodextrin, polyvinyl alcohol, povidone, propylene carbonate, propylene glycol alginate, sodium alginate, sodium starch glycolate, starch tragacanth, xanthan gum and combinations thereof.

Sweetening agents such as sorbitol, saccharin, sodium saccharin, sucrose, aspartame, fructose, mannitol, and invert sugar can be added to improve the taste.

Preservatives and chelating agents such as alcohol, sodium benzoate, butylated hydroxyl toluene, butylated hydroxyanisole, and ethylenediamine tetraacetic acid can be added at levels safe for ingestion to improve storage stability.

According to the present disclosure, a liquid composition can also contain a buffer such as gluconic acid, lactic acid, citric acid, or acetic acid, sodium gluconate, sodium lactate, sodium citrate, or sodium acetate. Selection of excipients and the amounts used can be readily determined by the formulation scientist based upon experience and consideration of standard procedures and reference works in the field.

The solid compositions of the present disclosure include powders, granulates, aggregates, and compacted compositions. The dosages include dosages suitable for oral, buccal, rectal, parenteral (including subcutaneous, intramuscular, and intravenous), inhalant, and ophthalmic administration. Although the most suitable administration in any given case will depend on the nature and severity of the condition being treated, in embodiments the route of administration is oral. The dosages can be conveniently presented in unit dosage form and prepared by any of the methods well-known in the pharmaceutical arts.

Dosage forms include solid dosage forms like tablets, powders, capsules, suppositories, sachets, troches, and lozenges, as well as liquid syrups, suspensions, and elixirs.

The dosage form of the present disclosure can be a capsule containing the composition, such as a powdered or granulated solid composition of the disclosure, within either a hard or soft shell. The shell can be made from gelatin and optionally contain a plasticizer such as glycerin and/or sorbitol, an opacifying agent and/or colorant.

The active ingredient and excipients can be formulated into compositions and dosage forms according to methods known in the art.

A composition for tableting or capsule filling can be prepared by wet granulation. In wet granulation, some or all of the active ingredients and excipients in powder form are blended and then further mixed in the presence of a liquid, typically water, that causes the powders to clump into granules. The granulate is screened and/or milled, dried, and then screened and/or milled to the desired particle size. The granulate can then be tableted, or other excipients can be added prior to tableting, such as a glidant and/or a lubricant.

A tableting composition can be prepared conventionally by dry blending. For example, the blended composition of the actives and excipients can be compacted into a slug or a sheet and then comminuted into compacted granules. The compacted granules can subsequently be compressed into a tablet.

As an alternative to dry granulation, a blended composition can be compressed directly into a compacted dosage form using direct compression techniques. Direct compression produces a more uniform tablet without granules. Excipients that are particularly well suited for direct compression tableting include microcrystalline cellulose, spray dried lactose, dicalcium phosphate dihydrate, and colloidal silica. The proper use of these and other excipients in direct compression tableting is known to those in the art with experience and skill in particular formulation challenges of direct compression tableting.

A capsule filling of the present disclosure can include any of the aforementioned blends and granulates that were described with reference to tableting, but they are not subjected to a final tableting step.

A pharmaceutical formulation of crystalline Trilaciclib citrate salt can be administered. Crystalline Trilaciclib citrate salt, may be formulated for administration to a mammal, in embodiments to a human, by injection. Crystalline Trilaciclib citrate salt can be formulated, for example, as a viscous liquid solution or suspension, such as a clear solution, for injection. The formulation can contain one or more solvents. A suitable solvent can be selected by considering the solvent's physical and chemical stability at various pH levels, viscosity (which would allow for syringeability), fluidity, boiling point, miscibility, and purity. Suitable solvents include alcohol USP, benzyl alcohol NF, benzyl benzoate USP, and Castor oil USP. Additional substances can be added to the formulation such as buffers, solubilizers, and antioxidants, among others. Ansel et al., Pharmaceutical Dosage Forms and Drug Delivery Systems, 7th ed.

The crystalline Trilaciclib citrate salt of the present disclosure, can be used as medicaments.

Having thus described the disclosure with reference to particular preferred embodiments and illustrative examples, those in the art can appreciate modifications to the disclosure as described and illustrated that do not depart from the scope of the disclosure as disclosed in the specification. The Examples are set forth to aid in understanding the disclosure but are not intended to, and should not be construed to limit its scope in any way.

### Powder X-ray Diffraction ("XRPD") method

X-ray diffraction was performed on X-Ray powder diffractometer:
Bruker D8 Advance; CuKα radiation (λ = 1.5418 Å); Lynx eye detector; laboratory temperature 22-25 °C; PMMA specimen holder ring with silicon low background. Prior to analysis, the samples were gently ground by means of mortar and pestle in order to obtain a fine powder. The ground sample was adjusted into a cavity of the sample holder and the surface of the sample was smoothed by means of a cover glass.

### Measurement parameters:

Scan range: 2 - 40 degrees 2-theta;
Scan mode: continuous;
Step size: 0.05 degrees;
Time per step: 0.5 s;
Sample spin: 30 rpm;
Sample holder: PMMA specimen holder ring with silicon low background.

All X-Ray Powder Diffraction peak values are calibrated with regard to standard silicon spiking in the sample.

### EXAMPLES

### Preparation of starting materials

Trilaciclib can be prepared according to methods known from the literature, for example U.S. Patent No. 8598186. Examples marked with an asterisk (*) are provided by way of reference only.

### *Example 1: Preparation of Trilaciclib Form TT1:

Trilaciclib (0.05 g) was dissolved in 7 mL Dichloromethane: Isopropanol (80:20, v/v) mixture at 50°C. The clear solution was cooled to 25°C and 3 mL of n-heptane were added under magnetic stirring at 25°C. The reaction mixture was maintained for about 20 hours at 25°C. Solid obtained was filtered and dried under vacuum for 10-15 minutes. The obtained solid was analyzed by XRD and designated as Form TT1 of Trilaciclib (Figure 1).

### *Example 2: Preparation of Trilaciclib Form TT2:

Trilaciclib (0.5 g) was dissolved in 4 mL acetic acid at 25-30°C. To the clear solution, 3 mL n-heptane were added and the reaction mixture was maintained under stirring for about 20 hours at 25°C. Solid obtained was filtered and dried under vacuum for 10-15 minutes. The obtained solid was analyzed by XRD and designated as Trilaciclib Form TT2 (Figure 2).

### *Example 3: Preparation of Trilaciclib Form TT3:

Trilaciclib (0.5 g) was dissolved in 4 mL formic acid at 25-30°C. To the clear solution 3 mL n-heptane were added and the reaction mixture was maintained under stirring for about 20 hours at 25°C. Solid obtained was filtered and dried under vacuum for 10-15 minutes. The obtained solid was analyzed by XRD and designated as Trilaciclib Form TT3 (Figure 3).

### *Example 4: Preparation of Trilaciclib Form TT4:

Trilaciclib (0.5 g) was dissolved in 4 mL propionic acid at 25-30°C. To the clear solution 3 mL n-heptane were added and the reaction mixture was maintained under stirring for about 20 hours at 25°C. Solid obtained was filtered and dried under vacuum for 10-15 minutes. The obtained solid was analyzed by XRD and designated as Trilaciclib Form TT4 (Figure 4).

### *Example 5: Preparation of Trilaciclib trifluoroacetate salt Form TTFA1:

Trilaciclib (0.5 g) was dissolved in 4 mL trifluoroacetic acid at 25-30°C. To the clear solution 3 mL acetone were added and the reaction mixture was maintained under stirring for about 20 hours at 25°C. Solid obtained was filtered and dried under vacuum for 10-15 minutes. The obtained solid was analyzed by XRD and designated as Trilaciclib trifluoroacetate salt Form TTFA1 (Figure 5).

### *Example 6: Preparation of Trilaciclib dihydrochloride salt Form THCl1:

Trilaciclib (1.0 g) was dissolved in 200 mL Dichloromethane: Methanol (85:15; v/v) mixture at 60°C. The solution was cooled to 25°C and 0.75mL (2 mole equivalents) of IPA-HCl solution (about 18% Hydrochloric acid in Isopropanol) was added. The reaction mixture was stirred at 25°C for about 20 hours. The reaction mass was filtered and dried under vacuum for 10-15 minutes. The obtained solid was analyzed by XRD and designated as Trilaciclib dihydrochloride salt Form THCl1 (Figure 6).

### *Example 7: Preparation of Trilaciclib hydrochloride acid salt Form THCl:

Trilaciclib (2.0 g) was dissolved in 400 mL Dichloromethane: Methanol (80:20; v/v) mixture at 60°C. The solution was cooled to 25°C and 0.8 mL (1 mole equivalent) of IPA-HCl solution (about 18% Hydrochloric acid in Isopropanol) was added. The reaction mixture was stirred at 25°C for about 20 hours. To the reaction mixture 50mL of acetone were added and stirred at 25°C for about 1 hour. The reaction mass was filtered and dried under vacuum for 15-30 minutes at 25°C. The obtained solid was analyzed by XRD and designated as Trilaciclib hydrochloride salt Form THCl (Figure 7).

### Example 8: Preparation of Trilaciclib citrate salt Form TCT1:

Trilaciclib (0.25 g) was dissolved in 50 mL Dichloromethane: Methanol (80:20; v/v) mixture at 60°C. Cooled the solution to 25°C and added 107 mg of citric acid anhydrous (1 mole equivalent). The reaction mixture was stirred at 25°C for about 20 hours. The reaction mixture was cooled to 5°C, 50mL of acetone were added and the mixture was stirred for about 1 hour at 5°C. The reaction mass was filtered (at 5°C) and dried under vacuum for 15-30 minutes at 25°C. The obtained solid analyzed by XRD and designated as Trilaciclib citrate salt Form TCT1 (Figure 8).

### *Example 9: Preparation of Trilaciclib sulphate salt Form TS1:

Trilaciclib (1.0 g) was dissolved in 200 mL Dichloromethane: Methanol (80:20; v/v) mixture at 60°C. The solution was cooled to 25°C and 0.25 mL of sulphuric acid solution (98% solution, 2 mole equivalent) was added. The reaction mixture was stirred at 25°C for about 20 hours. To the reaction mixture 50mL of acetone were added and the mixture was stirred at 25°C for about 1 hour. The reaction mass was filtered and dried under vacuum for 15-30 minutes at 25°C. The obtained solid was analyzed by XRD and designated as Trilaciclib sulphate salt Form TS1 (Figure 9).

### *Example 10: Preparation of Trilaciclib mesylate salt Form TMSA1:

Trilaciclib (0.25 g) was dissolved in 50 mL Dichloromethane: Methanol (80:20; v/v) mixture at 60°C. The solution was cooled to 25°C and 0.08 mL of methane sulphonic acid (2 mole equivalent) was added. The reaction mixture was stirred at 25°C for about 20 hours. The reaction mixture was cooled to 5°C, 50mL of acetone were added and the mixture was stirred for about 1 hour at 5°C. The reaction mass was filtered (5°C) and dried under vacuum for 15-30 minutes at 25°C. The obtained solid was analyzed by XRD and designated as Trilaciclib mesylate salt Form TMSA1 (Figure 10).

### *Example 11: Preparation of Trilaciclib besylate salt Form TBSA1:

Trilaciclib (0.25 g) was dissolved in 50 mL Dichloromethane: Methanol (80:20; v/v) mixture at 60°C. The solution was cooled to 25°C and 88 mg of benzene sulphonic acid (1 mole equivalent) was added. The reaction mixture was stirred at 25°C for about 20 hours. The reaction mixture was cooled to 5°C, 50mL of acetone were added and the reaction was stirred for about 1 hour at 5°C. The reaction mass was filtered (at 5°C) and dried under vacuum for 15-30 minutes at 25°C. The obtained solid was analyzed by XRD and designated as Trilaciclib besylate salt Form TBSA1 (Figure 11).

### *Example 12: Preparation of Trilaciclib dihydrochloride salt Form THCl2

Trilaciclib (5g) was stirred in a mixture of Dichloromethane: Methanol (80:20 v/v, 500 mL) at 25°C for 15minutes. A solution of hydrochloric acid in Isopropanol (about 18%, 4mL) was added into the slurry mass to obtain a clear solution. The reaction mixture was stirred at 25°C for about 3 hours. n-Heptane (1000mL) was added dropwise over a period of time of 30 minutes. The obtained slurry was stirred at 25°C for about 17 hours, filtered under vacuum over 90 minutes and dried at 45°C in vacuum tray oven for 2 hours. The obtained solid analyzed by XRD and designated as Trilaciclib dihydrochloride salt Form THCl2.

### *Example 13: Preparation of Trilaciclib dihydrochloride salt Form THCl2

Trilaciclib (0.045 g) was suspended in 10 mL Dichloromethane: Ethanol (70:30; v/v) mixture and stirred for 30 minutes at 25°C. A solution of hydrochloric acid in Isopropanol (about 18%, 0.05mL) was added into the slurry mass to obtain a clear solution. The reaction mixture was stirred about 4 hours at 25°C. The obtained solid was filtered, dried under vacuum for 10-15 minutes at 25°C and analyzed by XRD-Trilaciclib dihydrochloride salt Form THCl2.

### *Example 14: Preparation of Trilaciclib dihydrochloride salt Form THCl2

Trilaciclib (0.2g) was suspended in 20 mL of Dichloromethane: Methanol (80:20; v/v) mixture and stirred at 25°C for 15 minutes. A solution of hydrochloric acid in Isopropanol (about 18%, 0.16mL) was added into the slurry mass to obtain a clear solution. The reaction mixture was stirred at 25°C for about 30 minutes. n-Heptane (100mL) was added over 5 minutes. The obtained slurry mass was cooled to 5°C and stirred at 5°C for about 1 hour. The obtained slurry mass was filtered under vacuum at 5°C and dried at 25°C. The obtained solid was analyzed by XRD and designated as Trilaciclib dihydrochloride salt Form THCl2 (Figure 12).

### *Example 15: Preparation of Trilaciclib dihydrochloride salt Form THCl3

Trilaciclib (0.045 g) was suspended in 10 mL Dichloromethane:Dioxane (70:30; v/v) mixture and stirred for 30 minutes at 25°C. A solution of hydrochloric acid in Isopropanol (about 18%, 0.05mL) was added and the reaction mixture was stirred for 20 hours at 25°C, filtered and dried under vacuum for 10-15 minutes. The obtained solid analyzed by XRD and designated as Trilaciclib dihydrochloride salt Form THCl3 (Figure 13).

### Example 16: Preparation of Trilaciclib dihydrochloride salt Form THCl1

Trilaciclib (0.045 g) was suspended in 10 mL Dichloromethane: Isopropanol (70:30, v/v) mixture at 25°C. A solution of hydrochloric acid in Isopropanol (about 18%, 0.05mL) was added and the reaction mixture was stirred for 4 hours at 25°C, filtered and dried under vacuum for 10-15 minutes. The obtained solid analyzed by XRD- Trilaciclib dihydrochloride salt Form THCl1.

### *Example 17: Preparation of Trilaciclib dihydrochloride salt Form THCl4

Trilaciclib (0.045 g) was suspended in 10 mL Dichloromethane and stirred for 30 minutes at 25°C. A solution of hydrochloric acid in Isopropanol (about 18%, 0.05mL) was added and the reaction mixture was stirred at 25°C for about 20 hours. The reaction mass was filtered and dried under vacuum for 10-15 minutes at 25°C. The obtained solid was analyzed by XRD and designated as Trilaciclib dihydrochloride salt Form THCl4 (Figure 14).

### *Example 18: Preparation of Trilaciclib dihydrochloride salt Form THCl4

Trilaciclib (0.045 g) was suspended in 10 mL Dichloromethane: Acetone (70:30; v/v) mixture and stirred for 30 minutes at 25°C. A solution of hydrochloric acid in Isopropanol (about 18%, 0.05mL) was added and the reaction mixture was stirred at 25°C for about 20 hours. The reaction mass was filtered and dried under vacuum for 10-15 minutes at 25°C. The obtained solid analyzed by XRD and designated as Trilaciclib dihydrochloride salt Form THCl4.

### *Example 19: Preparation of Trilaciclib dihydrochloride salt Form THCl5

Trilaciclib (0.045 g) was suspended in 10 mL Acetonitrile and stirred for 30 minutes at 25°C. A solution of hydrochloric acid in Isopropanol (about 18%, 0.05mL) was added and the reaction mixture was stirred at 25°C for about 20 hours. The reaction mass was filtered and dried under vacuum for 10-15 minutes at 25°C. The obtained solid was analyzed by XRD and designated as Trilaciclib Form THCl5.
Trilaciclib dihydrochloride salt Form THCl5 (Figure 15).

### *Example 20: Preparation of Trilaciclib dihydrochloride salt Form THCl5

Trilaciclib (0.045 g) was suspended in 10 mL Dichloromethane: Acetonitrile (70:30; v/v) mixture and stirred for 30 minutes at 25°C. A solution of hydrochloric acid in Isopropanol (about 18%, 0.05mL) was added and the reaction mixture was stirred at 25°C for about 20 hours. The reaction mass was filtered and dried under vacuum for 10-15 minutes at 25°C. The obtained solid was analyzed by XRD and designated as Trilaciclib dihydrochloride salt Form THCl5.

### *Example 21: Preparation of Trilaciclib dihydrochloride salt Form THCl6

Trilaciclib (0.045 g) was suspended in 5 mL Acetonitrile at 0°C. A solution of hydrochloric acid in Isopropanol (about 18%, 0.05mL) was added and the reaction mixture was stirred at 0°C for about 20 hours. The reaction mass was filtered and dried under vacuum for 10-15 minutes at 25°C. The obtained solid was analyzed by XRD and designated as Trilaciclib dihydrochloride salt Form THCl6 (Figure 16).

### *Example 22: Preparation of Trilaciclib hydrobromide salt Form THBr1

Trilaciclib (0.25 g) was suspended in 50 mL Methanol at 25°C. A solution of hydrobromic acid in water (48%, 0.25mL) was added and the obtained clear solution was stirred at 25°C for about 30 minutes; suspension was formed. Methyl tert-butyl ether (50mL) was added and the reaction mixture was stirred at 25°C for about 60 minutes. The reaction mass was filtered and dried under vacuum for 10-15 minutes at 25°C. The obtained solid was analyzed by XRD and designated as Trilaciclib hydrobromide salt Form THBr1 (Figure 17).

### *Example 23: Preparation of Trilaciclib dihydrochloride salt Form THCl6

Trilaciclib dihydrochloride (THCl1*, 2.0 g) was dissolved in a mixture of methanol : water (70:30 (v/v); 100 mL) at 60°C. The solution was filtered to remove any undissolved particles and the clear solution was dropwise added into precooled methyl tert-butyl ether (300 ml) at about 0-5°C under stirring. The reaction mixture was maintained under stirring at 0-5°C for about 2-4 hour, filtered and dried under vacuum at 25°C for about 30-60 minutes. The obtained solid was further dried under vacuum at 60°C for 24 hours. After cooling to room temperature, the obtained solid was analyzed by XRD- Trilaciclib dihydrochloride salt Form THCl6.

## Claims

1. Crystalline Trilaciclib citrate salt.

2. A crystalline form of Trilaciclib citrate salt according to Claim 1, designated Form TCT1, which is **characterized by**: an X-ray powder diffraction pattern substantially as depicted in Figure 8; or an X-ray powder diffraction pattern having peaks at 6.1, 7.3, 11.3, 15.3 and 19.3 degrees 2-theta ± 0.2 degrees 2-theta.

3. Crystalline Form TCT1 of Trilaciclib citrate salt according to Claim 2, which is further **characterized by** an X-ray powder diffraction pattern having peaks at 6.1, 7.3, 11.3, 15.3 and 19.3 degrees 2-theta ± 0.2 degrees 2-theta, and also having any one, two, three or four additional peaks selected from 8.9, 9.3, 20.4 and 22.7 degrees 2-theta ± 0.2 degrees 2-theta.

4. Crystalline Form TCT1 of Trilaciclib citrate salt according to Claim 2 or Claim 3, which is **characterized by** an X-ray powder diffraction pattern having peaks at 6.1, 7.3, 8.9, 9.3, 11.3, 15.3, 19.3, 20.4, and 22.7 degrees 2-theta ± 0.2 degrees 2-theta.

5. Crystalline Trilaciclib citrate salt according to any of Claims 2, 3 or 4, which is polymorphically pure.

6. Crystalline Trilaciclib citrate salt according to any of Claims 2, 3, 4, or 5, which is substantially free of any other solid state forms of Trilaciclib or salts thereof.

7. Crystalline Trilaciclib citrate salt according to any of Claims 2, 3, 4, 5, or 6, containing about 20% (w/w) or less, about 10% (w/w) or less, about 5% (w/w) or less, about 2% (w/w) or less, about 1% (w/w) or less, or about 0% (w/w) of any other forms of Trilaciclib or salts thereof.

8. Use of a crystalline Trilaciclib citrate salt according to any of Claims 1-7 for preparing other crystalline forms of Trilaciclib, salts of Trilaciclib or crystalline forms thereof, or solvates of Trilaciclib or crystalline forms thereof.

9. Use of crystalline Trilaciclib citrate salt according to any of Claims 1-7 for preparing pharmaceutical compositions of Trilaciclib or Trilaciclib salts.

10. A pharmaceutical composition comprising crystalline Trilaciclib citrate salt according to any of Claims 1-7, and at least one pharmaceutically acceptable excipient.

11. Use of Trilaciclib citrate salt according to any of Claims 1-7 for the preparation of a pharmaceutical composition and/or formulation.

12. A process for preparing the pharmaceutical composition according to Claim 10, comprising combining crystalline Trilaciclib citrate salt according to any of Claims 1-7 with at least one pharmaceutically acceptable excipient.

13. Crystalline Trilaciclib citrate salt according to any of Claims 1-7, or a pharmaceutical composition according to Claim10, for use as a medicament.

14. Crystalline Trilaciclib citrate salt according to any of Claims 1-7, or a pharmaceutical composition according to Claim 10, for use as a chemo protective agent; in particular to reduce chemotherapy-induced bone marrow suppression in patients with small cell lung cancer (SCLC), non-small cell lung cancer (NSCLC), colorectal cancer, breast cancer, and bladder cancer.

15. Crystalline Trilaciclib citrate salt according to any of Claims 1-7, or a pharmaceutical composition according to Claim 10, for use as a chemo protective agent to reduce chemotherapy-induced bone marrow suppression in patients with: small cell lung cancer (SCLC), non-small cell lung cancer (NSCLC), metastatic colorectal cancer, metastatic triple-negative breast cancer, and advanced/metastatic bladder cancer; and preferably in patients with SCLC.

## Patentansprüche

1. Kristallines Trilaciclib-Citratsalz.

2. Kristalline Form von Trilaciclib-Citratsalz nach Anspruch 1, bezeichnet als Form TCT1, die **gekennzeichnet ist durch**: ein Röntgenpulverbeugungsmuster im Wesentlichen wie in Figur 8 dargestellt; oder ein Röntgenpulverbeugungsmuster mit Maxima bei 6,1, 7,3, 11,3, 15,3 und 19,3 Grad 2-Theta ± 0,2 Grad 2-Theta.

3. Kristalline Form TCT1 von Trilaciclib-Citratsalz nach Anspruch 2, die ferner **gekennzeichnet ist durch** ein Röntgenpulverbeugungsmuster mit Maxima bei 6,1 7,3, 11,3, 15,3 und 19,3 Grad 2-Theta ± 0,2 Grad 2-Theta und die ferner ein, zwei, drei oder vier zusätzliche Maxima ausgewählt aus 8,9, 9,3, 20,4 und 22,7 Grad 2-Theta ± 0,2 Grad 2-Theta aufweist.

4. Kristalline Form TCT1 von Trilaciclib-Citratsalz nach Anspruch 2 oder Anspruch 3, die **gekennzeichnet ist durch** ein Röntgenpulverbeugungsmuster mit Maxima bei 6,1, 7,3, 8,9, 9,3, 11,3, 15,3, 19,3, 20,4 und 22,7 Grad 2-Theta ± 0,2 Grad 2-Theta.

5. Kristallines Trilaciclib-Citratsalz nach einem der Ansprüche 2, 3 oder 4, das polymorphisch rein ist.

6. Kristallines Trilaciclib-Citratsalz nach einem der Ansprüche 2, 3, 4 oder 5, das im Wesentlichen frei von jeglichen anderen Festkörperformen von Trilaciclib oder Salzen davon ist.

7. Kristallines Trilaciclib-Citratsalz nach einem der Ansprüche 2, 3, 4, 5 oder 6, enthaltend etwa 20 % (w/w) oder weniger, etwa 10 % (w/w) oder weniger, etwa 5 % (w/w) oder weniger, etwa 2 % (w/w) oder weniger, etwa 1 % (w/w) oder weniger oder etwa 0 % (w/w) an jeglichen anderen Formen von Trilaciclib oder Salzen davon.

8. Verwendung eines kristallinen Trilaciclib-Citratsalzes nach einem der Ansprüche 1-7 zur Herstellung von anderen kristallinen Formen von Trilaciclib, Salzen von Trilaciclib oder kristallinen Formen davon oder Solvaten von Trilaciclib oder kristallinen Formen davon.

9. Verwendung von kristallinem Trilaciclib-Citratsalz nach einem der Ansprüche 1-7 zur Herstellung von pharmazeutischen Zusammensetzungen von Trilaciclib oder Trilaciclib-Salzen.

10. Pharmazeutische Zusammensetzung umfassend kristallines Trilaciclib-Citratsalz nach einem der Ansprüche 1-7 und wenigstens einen pharmazeutisch annehmbaren Hilfsstoff.

11. Verwendung von kristallinem Trilaciclib-Citratsalz nach einem der Ansprüche 1-7 zur Herstellung einer pharmazeutischen Zusammensetzung und/oder Formulierung.

12. Verfahren zur Herstellung der pharmazeutischen Zusammensetzung nach Anspruch 10, umfassend Kombinieren von kristallinem Trilaciclib-Citratsalz nach einem der Ansprüche 1-7 mit wenigstens einem pharmazeutisch annehmbaren Hilfsstoff.

13. Kristallines Trilaciclib-Citratsalz nach einem der Ansprüche 1-7 oder pharmazeutische Zusammensetzung nach Anspruch 10 zur Verwendung als Medikament.

14. Kristallines Trilaciclib-Citratsalz nach einem der Ansprüche 1-7 oder pharmazeutische Zusammensetzung nach Anspruch 10 zur Verwendung als chemoprotektives Mittel; insbesondere zum Verringern von chemotherapieinduzierter Knochenmarksuppression bei Patienten mit kleinzelligem Lungenkrebs (SCLC), nichtkleinzelligem Lungenkrebs (NSCLC), Kolorektalkrebs, Brustkrebs und Blasenkrebs.

15. Kristallines Trilaciclib-Citratsalz nach einem der Ansprüche 1-7 oder pharmazeutische Zusammensetzung nach Anspruch 10 zur Verwendung als chemoprotektives Mittel zum Verringern von chemotherapieinduzierter Knochenmarksuppression bei Patienten mit: kleinzelligem Lungenkrebs (SCLC), nichtkleinzelligem Lungenkrebs (NSCLC), metastasiertem Kolorektalkrebs, metastasiertem dreifach-negativem Brustkrebs und fortgeschrittenem/metastasiertem Blasenkrebs; und vorzugsweise bei Patienten mit SCLC.

## Revendications

1. Sel cristallin de citrate de Trilaciclib.

2. Forme cristalline de sel de citrate de Trilaciclib selon la revendication 1, désignée sous la forme TCT1, qui est **caractérisée par** : un diagramme de diffraction des rayons X sur poudre sensiblement tel que représenté sur la Figure 8 ; ou un diagramme de diffraction des rayons X sur poudre ayant des pics à 6,1, 7,3, 11,3, 15,3 et 19,3 degrés 2-thêta ± 0,2 degré 2-thêta.

3. Forme cristalline TCT1 de sel de citrate de Trilaciclib selon la revendication 2, qui est en outre **caractérisée par** un diagramme de diffraction des rayons X sur poudre ayant des pics à 6,1, 7,3, 11,3, 15,3 et 19,3 degrés 2-thêta ± 0,2 degré 2-thêta, et ayant également un, deux, trois ou quatre pics supplémentaires quelconques sélectionnés parmi 8,9, 9,3, 20,4 et 22,7 degrés 2-thêta ± 0,2 degré 2-thêta.

4. Forme cristalline TCT1 de sel de citrate de Trilaciclib selon la revendication 2 ou la revendication 3, qui est **caractérisée par** un diagramme de diffraction des rayons X sur poudre ayant des pics à 6,1, 7,3, 8,9, 9,3, 11,3, 15,3, 19,3, 20,4 et 22,7 degrés 2-thêta ± 0,2 degré 2-thêta.

5. Sel cristallin de citrate de Trilaciclib selon l'une quelconque des revendications 2, 3 ou 4, qui est polymorphiquement pur.

6. Sel cristallin de citrate de Trilaciclib selon l'une quelconque des revendications 2, 3, 4 ou 5, qui est sensiblement exempt de toute autre forme à l'état solide de Trilaciclib ou de sels de celui-ci.

7. Sel cristallin de citrate de Trilaciclib selon l'une quelconque des revendications 2, 3, 4, 5 ou 6, contenant environ 20 % (p/p) ou moins, environ 10 % (p/p) ou moins, environ 5 % (p/p) ou moins, environ 2 % (p/p) ou moins, environ 1 % (p/p) ou moins, ou environ 0 % (p/p) de toutes autres formes de Trilaciclib ou de sels de celui-ci.

8. Utilisation d'un sel cristallin de citrate de Trilaciclib selon l'une quelconque des revendications 1 à 7 pour la préparation d'autres formes cristallines de Trilaciclib, de sels de Trilaciclib ou de formes cristallines de ceux-ci, ou de solvates de Trilaciclib ou de formes cristallines de ceux-ci.

9. Utilisation de sel cristallin de citrate de Trilaciclib selon l'une quelconque des revendications 1 à 7 pour la préparation de compositions pharmaceutiques de Trilaciclib ou de sels de Trilaciclib.

10. Composition pharmaceutique comprenant un sel cristallin de citrate de Trilaciclib selon l'une quelconque des revendications 1 à 7 et au moins un excipient pharmaceutiquement acceptable.

11. Utilisation d'un sel de citrate de Trilaciclib selon l'une quelconque des revendications 1 à 7 pour la préparation d'une composition et/ou formulation pharmaceutique.

12. Procédé pour la préparation de la composition pharmaceutique selon la revendication 10, comprenant la combinaison d'un sel cristallin de citrate de Trilaciclib selon l'une quelconque des revendications 1 à 7 avec au moins un excipient pharmaceutiquement acceptable.

13. Sel cristallin de citrate de Trilaciclib selon l'une quelconque des revendications 1 à 7 ou composition pharmaceutique selon la revendication 10 pour une utilisation en tant que médicament.

14. Sel cristallin de citrate de Trilaciclib selon l'une quelconque des revendications 1 à 7, ou composition pharmaceutique selon la revendication 10, pour une utilisation comme agent chimio-protecteur ; en particulier pour réduire la suppression de moelle osseuse induite par chimiothérapie chez les patients atteints d'un cancer du poumon à petites cellules (SCLC), d'un cancer du poumon non à petites cellules (NSCLC), d'un cancer colorectal, d'un cancer du sein et d'un cancer de la vessie.

15. Sel cristallin de citrate de Trilaciclib selon l'une quelconque des revendications 1 à 7, ou composition pharmaceutique selon la revendication 10, pour une utilisation comme agent chimio-protecteur pour réduire la suppression de moelle osseuse induite par chimiothérapie chez les patients atteints de : cancer du poumon à petites cellules (SCLC), cancer du poumon non à petites cellules (NSCLC), cancer colorectal métastatique, cancer du sein triple négatif métastatique et cancer avancé/métastatique de la vessie ; et de préférence chez les patients atteints de SCLC.
